# EUROPEAN PATENT APPLICATION

(11) **EP 3 926 042 A1**
(43) Date of publication of application: **22.12.2021**
(21) Application number: 20755525.1
(22) Date of filing: 11.02.2020
(51) Int. Cl.: C12N 5/0781, C07K 14/705, A61P 35/00

(54) **ADJUVANT CAPABLE OF PROMOTING EXPANSION OF IMMUNE CELLS IN VIVO**

(30) Priority: 11.02.2019 CN 201910106097
(71) Applicant: Chineo Medical Technology Co., Ltd., Beijing 101111 (CN)
(72) Inventor: GU, Weiyue, Beijing 101111 (CN)
(74) Representative: Lavoix
(86) International application number: PCT/CN2020/074686
(87) International publication number: WO 2020/164465

(57) **Abstract**

The present disclosure provides an adjuvant that can boost the quantitative expansion of immune cells *in vivo,* and a combination comprising the adjuvant and immune cells. The present disclosure also provides a cascade booster system comprising the adjuvant and modified immune cells. The present disclosure also provides a treatment method using the adjuvant and the immune cells of the present disclosure.

## Description

### TECHNICAL FIELD

The present disclosure belongs to the bio-pharma field. Specifically, the present disclosure provides a booster adjuvant that can improve the expansion of immune cells including T cells *in vivo* to achieve quantitative expansion relatively controllable manually, thereby improving the effectiveness of immunotherapies.

### BACKGROUND

The primary obstacle in immune cell therapy against solid tumors is that a very small part of patient's autologous natural T cells can recognize tumor, which makes it very difficult to isolate tumor-recognizing T cells from autologous natural T cells. Secondly, even if they can be successfully isolated, it is still a very difficult process to expand them *in vitro* to a number required to fight against advanced tumors and produce significant therapeutic effects. Furthermore, even if they are expanded to a required number *in vitro,* the long cycle and high cost of this process pose a challenge to industrialization. In addition, many of the tumor-recognizing T cells are already in an exhausted state, and will die in large numbers after *in vitro* expansion, failing to exert the expected effect. How to improve the function of immune cells used in cell immunotherapy so as to achieve better therapeutic effects is an unresolved problem.

### SUMMARY OF THE INVENTION

The present invention provides a booster adjuvant for immune cell therapy, wherein the booster adjuvant can boost the expansion of the immune cells *in vivo* when it is used in combination with correspondingly modified immune cells, so that the expansion number is largely enhanced, thereby enhancing the effect of the immunotherapy and solving the above-mentioned problems.

The practice of some methods disclosed herein employ, unless otherwise indicated, conventional techniques of immunology, biochemistry, chemistry, molecular biology, microbiology, cell biology, genomics and recombinant DNA, which are within the skill of the art. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M.J. MacPherson, B.D. Hames and G.R. Taylor eds. (1995)), Harlow and Lane, eds. (1988) Antibodies, A Laboratory Manual, and Culture of Animal Cells: A Manual of Basic Technique and Specialized Applications, 6th Edition (R.I. Freshney, ed. (2010)).

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated, the term "about" meaning within an acceptable error range for the particular value should be assumed.

As used herein, a "cell" can generally refer to a biological cell. A cell can be the basic structural, functional and/or biological unit of a living organism. A cell can originate from any organism having one or more cells. Some non-limiting examples include: a prokaryotic cell, eukaryotic cell, a bacterial cell, an archaeal cell, a cell of a single-cell eukaryotic organism, a protozoa cell, a cell from a plant, an algal cell, a fungal cell, an animal cell, a cell from an invertebrate animal (e.g. fruit fly, cnidarian, echinoderm, nematode, etc.), a cell from a vertebrate animal (e.g., fish, amphibian, reptile, bird, mammal), a cell from a mammal (e.g., a pig, a cow, a goat, a sheep, a rodent, a rat, a mouse, a human, etc.), and etcetera. Sometimes a cell is not originating from a natural organism (e.g. a cell can be a synthetically made, sometimes termed an artificial cell).

The term "antigen," as used herein, refers to a molecule or a fragment thereof capable of being bound by a selective binding agent. As an example, an antigen can be a ligand that can be bound by a selective binding agent such as a receptor. As another example, an antigen can be an antigenic molecule that can be bound by a selective binding agent such as an immunological protein (e.g., an antibody). An antigen can also refer to a molecule or fragment thereof capable of being used in an animal to produce antibodies capable of binding to that antigen.

The term "neoantigen," as used herein, generally refers to tumor-specific antigens arising from mutations in a gene. The resulting mutated proteins, or fragments thereof, can trigger an antitumor T cell response.

The term "gene," as used herein, refers to a nucleic acid (e.g., DNA such as genomic DNA and cDNA) and its corresponding nucleotide sequence encoding an RNA transcript. The term as used herein with reference to genomic DNA includes intervening, non-coding regions as well as regulatory regions and can include 5' and 3' ends. In some uses, the term encompasses the transcribed sequences, including 5' and 3' untranslated regions (5'-UTR and 3'-UTR), exons and introns. In some genes, the transcribed region will contain "open reading frames" that encode polypeptides. In some uses of the term, a "gene" comprises only the coding sequences (e.g., an "open reading frame" or "coding region") necessary for encoding a polypeptide. In some cases, genes do not encode a polypeptide, for example, ribosomal RNA genes (rRNA) and transfer RNA (tRNA) genes. In some cases, the term "gene" includes not only the transcribed sequences, but in addition, also includes non-transcribed regions including upstream and downstream regulatory regions, enhancers and promoters. A gene can refer to an "endogenous gene" or a native gene in its natural location in the genome of an organism. A gene can refer to an "exogenous gene" or a non-native gene. A non-native gene can refer to a gene not normally found in the host organism but which is introduced into the host organism by gene transfer. A non-native gene can also refer to a gene not in its natural location in the genome of an organism. A non-native gene can also refer to a naturally occurring nucleic acid or polypeptide sequence that comprises mutations, insertions and/or deletions (e.g., non-native sequence).

The term "antibody," as used herein, refers to a proteinaceous binding molecule with immunoglobulin-like functions. The term antibody includes antibodies (e.g., monoclonal and polyclonal antibodies), as well as derivatives, variants, and fragments thereof. Antibodies include, but are not limited to, immunoglobulins (Ig) of different classes (i.e. IgA, IgG, IgM, IgD and IgE) and subclasses (such as IgG1, IgG2, etc.). A derivative, variant or fragment thereof can refer to a functional derivative or fragment which retains the binding specificity (e.g., complete and/or partial) of the corresponding antibody. Antigen-binding fragments include Fab, Fab', F(ab')2, variable fragment (Fv), single chain variable fragment (scFv), minibodies, diabodies, and single-domain antibodies ("sdAb" or "nanobodies" or "camelids"). The term antibody includes antibodies and antigen-binding fragments of antibodies that have been optimized, engineered or chemically conjugated. Examples of antibodies that have been optimized include affinity-matured antibodies. Examples of antibodies that have been engineered include Fc optimized antibodies (e.g., antibodies optimized in the fragment crystallizable region) and multispecific antibodies (e.g., bispecific antibodies).

The term "nucleotide," as used herein, generally refers to a base-sugar-phosphate combination. A nucleotide can comprise a synthetic nucleotide. A nucleotide can comprise a synthetic nucleotide analog. Nucleotides can be monomeric units of a nucleic acid sequence (e.g. deoxyribonucleic acid (DNA) and ribonucleic acid (RNA)). The term nucleotide can include ribonucleoside triphosphates adenosine triphosphate (ATP), uridine triphosphate (UTP), cytosine triphosphate (CTP), guanosine triphosphate (GTP) and deoxyribonucleoside triphosphates such as dATP, dCTP, dITP, dUTP, dGTP, dTTP, or derivatives thereof. Such derivatives can include, for example, [αS]dATP, 7-deaza-dGTP and 7-deaza-dATP, and nucleotide derivatives that confer nuclease resistance on the nucleic acid molecule containing them. The term nucleotide as used herein can refer to dideoxyribonucleoside triphosphates (ddNTPs) and their derivatives. Illustrative examples of dideoxyribonucleoside triphosphates can include, but are not limited to, ddATP, ddCTP, ddGTP, ddITP, and ddTTP. A nucleotide can be unlabeled or detectably labeled by well-known techniques. Labeling can also be carried out with quantum dots. Detectable labels can include, for example, radioactive isotopes, fluorescent labels, chemiluminescent labels, bioluminescent labels and enzyme labels.

The terms "polynucleotide," "oligonucleotide," and "nucleic acid" are used interchangeably to refer to a polymeric form of nucleotides of any length, either deoxyribonucleotides or ribonucleotides, or analogs thereof, either in single-, double-, or multi-stranded form. A polynucleotide can be exogenous or endogenous to a cell. A polynucleotide can exist in a cell-free environment. A polynucleotide can be a gene or fragment thereof. A polynucleotide can be DNA. A polynucleotide can be RNA. A polynucleotide can have any three dimensional structure, and can perform any function, known or unknown. A polynucleotide can comprise one or more analogs (e.g. altered backbone, sugar, or nucleobase).

The term "expression" refers to one or more processes by which a polynucleotide is transcribed from a DNA template (such as into an mRNA or other RNA transcript) and/or the process by which a transcribed mRNA is subsequently translated into peptides, polypeptides, or proteins. Transcripts and encoded polypeptides can be collectively referred to as "gene product." If the polynucleotide is derived from genomic DNA, expression can include splicing of the mRNA in a eukaryotic cell. "Up-regulated," with reference to expression, generally refers to an increased expression level of a polynucleotide (e.g., RNA such as mRNA) and/or polypeptide sequence relative to its expression level in a wild-type state while "down-regulated" generally refers to a decreased expression level of a polynucleotide (e.g., RNA such as mRNA) and/or polypeptide sequence relative to its expression in a wild-type state.

The term "regulating" with reference to expression or activity, as used herein, refers to altering the level of expression or activity. Regulation can occur at the transcription level and/or translation level.

The terms "peptide," "polypeptide," and "protein" are used interchangeably herein to refer to a polymer of at least two amino acid residues joined by peptide bond(s). This term does not connote a specific length of polymer, nor is it intended to imply or distinguish whether the peptide is produced using recombinant techniques, chemical or enzymatic synthesis, or is naturally occurring. The terms apply to naturally occurring amino acid polymers as well as amino acid polymers comprising at least one modified amino acid. In some cases, the polymer can be interrupted by non-amino acids. The terms include amino acid chains of any length, including full length proteins, and proteins with or without secondary and/or tertiary structure (e.g., domains). The terms also encompass an amino acid polymer that has been modified, for example, by disulfide bond formation, glycosylation, lipidation, acetylation, phosphorylation, oxidation, and any other manipulation such as conjugation with a labeling component. The terms "amino acid" and "amino acids," as used herein, generally refer to natural and non-natural amino acids, including, but not limited to, modified amino acids and amino acid analogues. Modified amino acids can include natural amino acids and non-natural amino acids, which have been chemically modified to include a group or a chemical moiety not naturally present on the amino acid. Amino acid analogues can refer to amino acid derivatives. The term "amino acid" includes both D-amino acids and L-amino acids.

The terms "derivative," "variant," and "fragment," when used herein with reference to a polypeptide, refers to a polypeptide related to a wild type polypeptide, for example either by amino acid sequence, structure (e.g., secondary and/or tertiary), activity (e.g., enzymatic activity) and/or function. Derivatives, variants and fragments of a polypeptide can comprise one or more amino acid variations (e.g., mutations, insertions, and deletions), truncations, modifications, or combinations thereof compared to a wild type polypeptide.

As used herein, "fusion" can refer to a protein and/or nucleic acid comprising one or more non-native sequences (e.g., moieties). A fusion can comprise one or more of the same non-native sequences. A fusion can comprise one or more of different non-native sequences. A fusion can be a chimera. A fusion can comprise a nucleic acid affinity tag. A fusion can comprise a barcode. A fusion can comprise a peptide affinity tag. A fusion can provide for subcellular localization of the site-directed polypeptide (e.g., a nuclear localization signal (NLS) for targeting to the nucleus, a mitochondrial localization signal for targeting to the mitochondria, a chloroplast localization signal for targeting to a chloroplast, an endoplasmic reticulum (ER) retention signal, and the like). A fusion can provide a non-native sequence (e.g., affinity tag) that can be used to track or purify. A fusion can be a small molecule such as biotin or a dye such as Alexa fluor dyes, Cyanine3 dye, Cyanine5 dye.

The phrase "artificial TCR" as used herein can be understood as "exogenous T cell receptor (TCR) complex", which refers to a TCR complex in which one or more chains of the TCR are introduced into the genome of an immune cell that may or may not endogenously express the TCR. In some cases, an exogenous TCR complex can refer to a TCR complex in which one or more chains of an endogenous TCR complex have one or more mutated sequences, for example at either the nucleic acid or amino acid level. Expression of an exogenous TCR on an immune cell can confer binding specificity for an epitope or antigen (e.g., an epitope or antigen preferentially present on the surface of a cancer cell or other disease-causing cell or particle). An exogenous TCR complex can comprise a TCR-alpha, a TCR-beta chain, a CD3-gamma chain, a CD3- delta chain, a CD3-zeta chain, or any combination thereof, which is introduced into the genome. In some cases, the chain introduced into the genome may replace the endogenously occurring chain.

The terms "subject," "individual," and "patient" are used interchangeably herein to refer to a vertebrate, preferably a mammal such as a human. Mammals include, but are not limited to, murines, simians, humans, farm animals, sport animals, and pets. Tissues, cells and their progeny of a biological entity obtained *in vivo* or cultured *in vitro* are also encompassed.

The term "treatment/treating" as used herein, refer to an approach for obtaining beneficial or desired results including but not limited to a therapeutic benefit and/or a prophylactic benefit. For example, a treatment can comprise administering an booster adjuvant and immune cells disclosed herein. By therapeutic benefit is meant any therapeutically relevant improvement in or effect on one or more diseases, conditions, or symptoms under treatment. For prophylactic benefit, a composition can be administered to a subject at risk of developing a particular disease, condition, or symptom, or to a subject reporting one or more of the physiological symptoms of a disease, even though the disease, condition, or symptom may not have yet been manifested.

The term "effective amount" or "therapeutically effective amount" refers to the quantity of a composition, for example a composition comprising booster adjuvant and/or immune cells such as lymphocytes (e.g., T lymphocytes and/or NK cells) of the present disclosure, that is sufficient to result in a desired activity upon administration to a subject in need thereof. Within the context of the present disclosure, the term "therapeutically effective" refers to that quantity of a composition that is sufficient to delay the manifestation, arrest the progression, relieve or alleviate at least one symptom of a disorder treated by the methods of the present disclosure.

The term "genetic profile," as used herein, refers to information about specific genes, including variations and gene expression in an individual or in a certain type of tissue. A genetic profile can be used for neoantigen selection. The term "somatic mutation profile," as used herein, refers to information about specific genes associated with somatic mutation, including but not limited to specific genes resulted from somatic mutation. A somatic mutation profile can be used for neoantigen selection.

The term "booster" or "adjuvant" as used in the present disclosure refers to an agent that can assist immune cell therapy, and is characterized by having a booster antigen (BA), wherein the booster antigen is a specific antigen that can be specifically recognized by a specific therapeutic immune cell, and the adjuvant can stimulate and boost the immune cells to expand quantitatively *in vivo* via the booster antigen.

In some cases, a certain therapeutic immune cell recognizes the booster antigen by expressing an artificially modified chimeric antigen receptor (CAR) on the therapeutic immune cell, and the CAR is used to specifically recognize the booster antigen. The CAR is a chimeric antigen receptor targeting a Booster Antigen (hereinafter may be referred to as "anti-Booster Antigen-CAR" or "aBA-CAR"), and its structure contains an antigen-binding domain, such as a variable region, of the antibody that recognizes the booster antigen.

In some embodiments, the booster antigen can be a variant of membrane protein widely expressed by human cells, or a variant of membrane protein expressed by tumor cells, wherein the membrane protein comprises a class I RTK (e.g., the epidermal growth factor (EGF) receptor family including EGFR; the ErbB family including ErbB-2, ErbB-3, and ErbB-4), a class II RTK (e.g., the insulin receptor family including INSR, IGF-1R, and IRR), a class III RTK (e.g., the platelet-derived growth factor (PDGF) receptor family including PDGFR-α, PDGFR-β, CSF-1R, KIT/SCFR, and FLK2/FLT3), a class IV RTK (e.g., the fibroblast growth factor (FGF) receptor family including FGFR-1, FGFR-2, FGFR-3, and FGFR-4), a class V RTK (e.g., the vascular endothelial growth factor (VEGF) receptor family including VEGFR1, VEGFR2, and VEGFR3), a class VI RTK (e.g., the hepatocyte growth factor (HGF) receptor family including hepatocyte growth factor receptor (HGFR/MET) and RON), a class VII RTK (e.g., the tropomyosin receptor kinase (Trk) receptor family including TRKA, TRKB, and TRKC), a class VIII RTK (e.g., the ephrin (Eph) receptor family including EPHA1, EPHA2, EPHA3, EPHA4, EPHA5, EPHA6, EPHA7, EPHA8, EPHB1, EPHB2, EPHB3, EPHB4, EPHB5, and EPHB6), a class IX RTK (e.g., AXL receptor family such as AXL, MER, and TRYO3), a class X RTK (e.g., LTK receptor family such as LTK and ALK), a class XI RTK (e.g., TIE receptor family such as TIE and TEK), a class XII RTK (e.g., ROR receptor family ROR1 and ROR2), a class XIII RTK (e.g., the discoidin domain receptor (DDR) family such as DDR1 and DDR2), a class XIV RTK (e.g., RET receptor family such as RET), a class XV RTK (e.g., KLG receptor family including PTK7), a class XVI RTK (e.g., RYK receptor family including Ryk), a class XVII RTK (e.g., MuSK receptor family such as MuSK), CD47, CD70, NKG2D or any derivative, variant or fragment thereof.

In some embodiments, the booster antigen can comprise at least an extracellular region (e.g., ligand binding domain) of a catalytic receptor such as a receptor tyrosine kinase (RTK), or any derivative, variant or fragment thereof. In some embodiments, the booster antigen comprises a class I RTK (e.g., the epidermal growth factor (EGF) receptor family including EGFR; the ErbB family including ErbB-2, ErbB-3, and ErbB-4), a class II RTK (e.g., the insulin receptor family including INSR, IGF-1R, and IRR), a class III RTK (e.g., the platelet-derived growth factor (PDGF) receptor family including PDGFR-α, PDGFR-β, CSF-1R, KIT/SCFR, and FLK2/FLT3), a class IV RTK (e.g., the fibroblast growth factor (FGF) receptor family including FGFR-1, FGFR-2, FGFR-3, and FGFR-4), a class V RTK (e.g., the vascular endothelial growth factor (VEGF) receptor family including VEGFR1, VEGFR2, and VEGFR3), a class VI RTK (e.g., the hepatocyte growth factor (HGF) receptor family including hepatocyte growth factor receptor (HGFR/MET) and RON), a class VII RTK (e.g., the tropomyosin receptor kinase (Trk) receptor family including TRKA, TRKB, and TRKC), a class VIII RTK (e.g., the ephrin (Eph) receptor family including EPHA1, EPHA2, EPHA3, EPHA4, EPHA5, EPHA6, EPHA7, EPHA8, EPHB1, EPHB2, EPHB3, EPHB4, EPHB5, and EPHB6), a class IX RTK (e.g., AXL receptor family such as AXL, MER, and TRYO3), a class X RTK (e.g., LTK receptor family such as LTK and ALK), a class XI RTK (e.g., TIE receptor family such as TIE and TEK), a class XII RTK (e.g., ROR receptor family ROR1 and ROR2), a class XIII RTK (e.g., the discoidin domain receptor (DDR) family such as DDR1 and DDR2), a class XIV RTK (e.g., RET receptor family such as RET), a class XV RTK (e.g., KLG receptor family including PTK7), a class XVI RTK (e.g., RYK receptor family including Ryk), a class XVII RTK (e.g., MuSK receptor family such as MuSK), CD47, CD70, NKG2D or any derivative, variant or fragment thereof.

A booster antigen can comprise a RTK, or any derivative, variant or fragment thereof. Non limiting examples of RTK ligands include growth factors, cytokines, and hormones. Growth factors include, for example, members of the epidermal growth factor family (e.g., epidermal growth factor or EGF, heparin-binding EGF-like growth factor or HB-EGF, transforming growth factor-a or TGF-α, amphiregulin or AR, epiregulin or EPR, epigen, betacellulin or BTC, neuregulin-1 or NRG1, neuregulin-2 or NRG2, neuregulin-3 or NRG3, and neuregulin-4 or NRG4), the fibroblast growth factor family (e.g., FGF1, FGF2, FGF3, FGF4, FGF5, FGF6, FGF7, FGF8, FGF9, FGF10, FGF11, FGF12, FGF13, FGF14, FGF15/19, FGF16, FGF17, FGF18, FGF20, FGF21, and FGF23), the vascular endothelial growth factor family (e.g., VEGF-A, VEGF-B, VEGF-C, VEGF-D, and PIGF), and the platelet-derived growth factor family (e.g., PDGFA, PDGFB, PDGFC, and PDGFD). Hormones include, for example, members of the insulin/IGF/relaxin family (e.g., insulin, insulin-like growth factors, relaxin family peptides including relaxin1, relaxin2, relaxin3, Leydig cell-specific insulin-like peptide (gene INSL3), early placenta insulin-like peptide (ELIP) (gene INSL4), insulin-like peptide 5 (gene INSL5), and insulin-like peptide 6).

In some embodiments, a booster antigen comprises at least an extracellular region (e.g., ligand binding domain) of a catalytic receptor such as a receptor threonine/serine kinase (RTSK), or any derivative, variant or fragment thereof, or comprises a fragment of variable region of an antibody with such fragment as antigen. A booster antigen can comprise a type I RTSK, type II RTSK, or any derivative, variant or fragment thereof. A booster antigen can comprise a type I receptor, or any derivative, variant or fragment thereof, selected from the group consisting of: ALK1 (ACVRL1), ALK2 (ACVR1A), ALK3 (BMPR1A), ALK4 (ACVR1B), ALK5 (TGFβR1), ALK6 (BMPR1B), and ALK7 (ACVR1C). A booster antigen can comprise a type II receptor, or any derivative, variant or fragment thereof, selected from the group consisting of: TGFβR2, BMPR2, ACVR2A, ACVR2B, and AMHR2 (AMHR). In some embodiments, the booster antigen comprises a TGF-β receptor.

A booster antigen comprises a RTSK, or any derivative or variant thereof.

In some cases, a booster antigen is B cell surface protein or fragment thereof. The B cell surface protein can be any protein that may be found on the surface of a B cell. Non-limiting examples include CD1d, CD5, CD10, CD11a, CD19, CD20, CD21, CD22, CD23, CD24, CD25, CD27, CD28, CD29, CD34, CD37, CD38, CD40, CD44, CD45, CD49b, CD69, CD72, CD74, CD80, CD83, CD84, CD86, CD93, CD95, CD117, CD127, CD138, CD147, CD148, CD185, CD270, CD284, and CD360. In some embodiments, the antigen interacting domain of the CAR can be capable of binding a surface protein on a non-B cell, as long as the binding to the surface protein does not significantly compromise the general health status or the immune system of the host. In some embodiments, the surface protein is a surface protein on an immune cell. In some embodiments, the surface protein is a surface protein on a cell other than an immune cell. In some embodiments, the surface protein may be selected from, with the proviso that the binding to the surface protein does not significantly compromise the general health status or the immune system of the host, CD31, CD32 (A, B), CD33, CD34, CD35, CD36, CD37, CD38, CD39, CD40, CD41, CD42(a, b, c, d), CD43, CD44, CD45, CD46, CD47, CD48, CD49 (a, b, c, d, e, f), CD50, CD51, CD52, CD53, CD54, CD55, CD56, CD57, CD58, CD59, CD61, CD62 (E, L, P), CD63, CD64 (A, B, C), CD66 (a, b, c, d, e, f), CD68, CD69, CD70, CD71, CD72, CD73, CD74, CD78, CD79 (a, b), CD80, CD81, CD82, CD83, CD84, CD85 (a, d, e, h, j, k), CD86, CD87, CD88, CD89, CD90, CD91, CD92, CD93, CD94, CD95, CD96, CD97, CD98, CD99, CD100, CD1 (a-c), 1A, ID, IE, CD2, CD3 (γ, δ, ε), CD4, CD5, CD6, CD7, CD8, a, CD9, CD10, CD11 (a, b, c, d), CD13, CD14, CD15, CD16, A, B, CD18, CD19, CD20, CD21, CD22, CD23, CD24, CD25, CD26, CD27, CD28, CD29, CD30, CD101, CD102, CD103, CD104, CD105, CD106, CD107 (a, b), CD108, CD109, CD110, CD111, CD112, CD113, CD114, CD115, CD116, CD117, CD118, CD119, CD120 (a, b), CD121 (a, b), CD122, CD123, CD124, CD125, CD126, CD127, CD129, CD130, CD131, CD132, CD133, CD134, CD135, CD136, CD137, CD138, CD140b, CD141, CD142, CD143, CD144, CD146, CD147, CD148, CD150, CD191, CD192, CD193, CD194, CD195, CD196, CD197, CDw198, CDw199, CD200, CD201, CD202b, CD204, CD205, CD206, CD207, CD208, CD209, CDw210 (a, b), CD212, CD213a (1, 2), CD217, CD218, (a, b), CD220, CD221, CD222, CD223, CD224, CD225, CD226, CD227, CD228, CD229, CD230, CD233, CD234, CD235 (a, b), CD236, CD238, CD239, CD240CE, CD240D, CD241, CD243, CD244, CD246, CD247, CD248, CD249, CD252, CD253, CD254, CD256, CD257, CD258, CD261, CD262, CD263, CD264, CD265, CD266, CD267, CD268, CD269, CD271, CD272, CD273, CD274, CD275, CD276, CD278, CD279, CD280, CD281, CD282, CD283, CD284, CD286, CD288, CD289, CD290, CD292, CDw293, CD294, CD295, CD297, CD298, CD299, CD300A, CD301, CD302, CD303, CD304, CD305, CD306, CD307, CD309, CD312, CD314, CD315, CD316, CD317, CD318, CD320, CD321, CD322, CD324, CD325, CD326, CD328, CD329, CD331, CD332, CD333, CD334, CD335, CD336, CD337, CD338, CD339, CD340, CD344, CD349, CD350, CD151, CD152, CD153, CD154, CD155, CD156 (a, b, c), CD157, CD158, (a, d, e, i, k), CD159 (a, c), CD160, CD161, CD162, CD163, CD164, CD166, CD167 (a, b), CD168, CD169, CD170, CD171, CD172 (a, b, g), CD174, CD177, CD178, CD179 (a, b), CD180, CD181, CD182, CD183, CD184, CD185, and CD186.

In some cases, the booster antigen is a full length surface protein of NK cells, granulocytes, red blood cells, or platelets, or a fragment thereof.

In some cases, the booster antigen is a surface marker or transmembrane protein or a fragment thereof of a pathogen such as viruses, bacteria or fungi, wherein said pathogen includes HPV virus, EB virus, Helicobacter pylori (HP), hepatitis B virus (HBV), and HIV (HIV).

In some cases, the booster antigen is any kind of human intracellular protein or a fragment thereof.

In some cases, the booster antigen is any kind of human extracellular secretion protein or a fragment thereof.

In some embodiments, the booster antigen is a wild-type or variant of a complete human natural protein or a partial fragment thereof, or a combination of wild-type or variant of a plurality of complete natural human proteins or fragments thereof.

In some embodiments, the booster antigen is CD19 or EGFRviii (including complete EGFRviii or EGFRviiit, i.e. truncated EGFRviii), or is a composition comprising CD19 and/or CD86 and/or CD137L and/or mbIL15 (membrane-bound IL15) and/or IL15Ra (α subunit of IL15 receptor), or is a composition comprising EGFRviii and/or CD86 and/or CD137L and/or mbIL15 and/or IL15Ra.

In some cases, the booster adjuvant can be a protein, a compound, a complex, a cell (or "booster cell" hereinafter) or artificial nanomaterial comprising the booster antigen.

In some embodiments, the booster antigen can be coupled (e.g., via covalent and/or non-covalent bonds) to the surface of particles (e.g., nanoparticles). The particles may be any particulate materials comprising organic and/or inorganic material. The particles may have various shapes and sizes. The particles may be about 1 nanometers (nm) to about 50 micrometers (µm) in at least one dimension. The particles may be at least about 1 nm, 5 nm, 10 nm, 50 nm, 100 nm, 500 nm, 1 µm, 5 µm, 10 µm, 50 µm, or more in at least one dimension. The particles may be at most about 50 µm, 10 µm, 5 µm, 1 µm, 500 nm, 100 nm, 50 nm, 10 nm, 5 nm, 1 nm, or less in at least one dimension. The particles may be nanoparticles, microparticles, nanospheres, micropsheres, nanorods, microrods, nanofibers, nanoribbons, etc. Examples of the particle include metal nanoparticles (e.g., gold nanoparticles, silver nanoparticles, and iron nanoparticles), intermetallic nanosemiconductor nanoparticles, core-shell nanoparticles, particles with an inorganic core with a polymer shell, particles with an organic core with a polymer shell, and mixtures thereof. Alternatively, the particles can be organic nanoparticles, such as crosslinked polymers, hydrogel polymers, biodegradable polymers, polylactide (PLA), polyglycolide (PGA), polycaprolactone (PCL), copolymers, polysaccharides, starch, cellulose, chitosan, polyhydroxyalkanoates (PHA), PHB, PHV, lipids, peptides, peptide amphiphiles, polypeptides (e.g., proteins), or combinations thereof. The particles presenting the B cell surface protein on the surface may be introduced *in vitro* to the immune cell comprising the CAR that binds the B cell surface protein. Alternatively or in addition to, the particles presenting the B cell surface protein may be introduced *in vivo* (e.g., local or systemic injection) along with the immune cell comprising the CAR. Such particles may be used to expand the population of the immune cell comprising the CAR *in vitro* or *in vivo.*

In some cases, the booster adjuvant is a booster cell, and the booster cell is derived from any type of cells or a mixture of various types of cells such as a patient's autologous or heterologous peripheral blood mononuclear cells (PBMC) or B cells, T cells, and NK cells, wherein the cells can be unmodified natural cells, or modified cells, or immortalized B cells or NK cells (such as NK92) or K562 cells.

In some cases, the booster adjuvant is a booster cell (such as a T cell), with a transmembrane booster antigen is expressed on the booster cell, wherein the booster antigen is a fusion protein consisted of an extracellular binding domain and a transmembrane region, wherein the extracellular binding domain is a structure that can be recognized by a specific therapeutic immune cell (such as EGFRviii or CD19), and the transmembrane region is a part or all of the structure of a protein naturally expressed by the booster cell (such as CD3).

"Antigen-binding domain" of aBA-CAR can comprise any protein or molecule capable of binding to a booster antigen. Non-limiting examples of the antigen binding domain include, but are not limited to, a monoclonal antibody, a polyclonal antibody, a recombinant antibody, a human antibody, a humanized antibody, a murine antibody, or a functional derivative, variant or fragment thereof, including, but not limited to, a Fab, a Fab', a F(ab')2, an Fv, a single-chain Fv (scFv), minibody, a diabody, and a single-domain antibody such as a heavy chain variable domain (VH), a light chain variable domain (VL) and a variable domain (VHH) of camelid derived nanobody. In some embodiments, the antigen binding domain comprises at least one of a Fab, a Fab', a F(ab')2, an Fv, and a scFv. In some embodiments, the antigen binding domain comprises an antibody mimetic. Antibody mimetics refer to molecules which can bind a target molecule with an affinity comparable to an antibody, and include single-chain binding molecules, cytochrome b562-based binding molecules, fibronectin or fibronectin-like protein scaffolds (e.g., adnectins), lipocalin scaffolds, calixarene scaffolds, A-domains and other scaffolds. In some embodiments, an antigen binding domain comprises a transmembrane receptor, or any derivative, variant, or fragment thereof. For example, an antigen binding domain can comprise at least a ligand binding domain of a transmembrane receptor.

In some embodiments, the "antigen binding domain" of aBA-CAR can comprise a scFV. A scFv can be derived from an antibody for which the sequences of the variable regions are known. In some embodiments, a scFv can be derived from an antibody sequence obtained from an available mouse hybridoma. A scFv can be obtained from whole-exomic sequencing of a tumor cell or primary cell. In some embodiments, a scFv can be altered. For instance, a scFv may be modified in a variety of ways. In some cases, a scFv can be mutated, so that the scFv may have higher affinity to its target. In some cases, the affinity of the scFv for its target can be optimized for targets expressed at low levels on normal tissues. This optimization can be performed to minimize potential toxicities, such as hypercytokinemia. In other cases, the cloning of a scFv that has a higher affinity for the membrane bound form of a target can be preferable over its soluble form counterpart. This modification can be performed if some targets can also be detected in soluble form at different levels and their targeting can cause unintended toxicity, such as hypercytokinemia.

The antigen binding domain of a aBA-CAR of a subject system can be linked to the intracellular signaling domain via a transmembrane domain. A transmembrane domain can be a membrane spanning segment. A transmembrane domain of a subject CAR can anchor the CAR to the plasma membrane of a cell, for example an immune cell. In some embodiments, the membrane spanning segment comprises a polypeptide. The membrane spanning polypeptide linking the antigen binding domain and the intracellular signaling domain of the CAR can have any suitable polypeptide sequence. In some cases, the membrane spanning polypeptide comprises a polypeptide sequence of a membrane spanning portion of an endogenous or wild-type membrane spanning protein. In some embodiments, the membrane spanning polypeptide comprises a polypeptide sequence having at least 1 (e.g., at least 2, 3, 4, 5, 6, 7, 8, 9, 10 or greater) of an amino acid substitution, deletion, and insertion compared to a membrane spanning portion of an endogenous or wild-type membrane spanning protein. In some embodiments, the membrane spanning polypeptide comprises a non-natural polypeptide sequence, such as the sequence of a polypeptide linker. The polypeptide linker may be flexible or rigid. The polypeptide linker can be structured or unstructured. In some embodiments, the membrane spanning polypeptide transmits a signal from an extracellular region of a cell to an intracellular region via the antigen binding domain. A native transmembrane portion of CD28 can be used in a aBA-CAR. In other cases, a native transmembrane portion of CD8 alpha can also be used in a aBA-CAR.

A CAR of the present disclosure, including an aBA-CAR, can comprise a signaling domain, or any derivative, variant, or fragment thereof, involved in immune cell signaling. The intracellular signaling domain of a CAR can induce activity of an immune cell comprising the CAR. The intracellular signaling domain can transduce the effector function signal and direct the cell to perform a specialized function. The signaling domain can comprise signaling domains of other molecules. In some cases, a truncated portion of the signaling domain is used in a CAR.

In some embodiments, the intracellular signaling domain comprises multiple signaling domains involved in immune cell signaling, or any derivatives, variants, or fragments thereof. For example, the intracellular signaling domain can comprise at least 2 immune cell signaling domains, e.g., at least 2, 3, 4, 5, 7, 8, 9, or 10 immune cell signaling domains. An immune cell signaling domain can be involved in regulating primary activation of the TCR complex in either a stimulatory way or an inhibitory way. The intracellular signaling domain may be that of a T-cell receptor (TCR) complex. The intracellular signaling domain of a subject CAR can comprise a signaling domain of an Fcγ receptor (FcyR), an Fcε receptor (FcεR), an Fcα receptor (FcaR), neonatal Fc receptor (FcRn), CD3, CD3 ζ, CD3 γ, CD3 δ, CD3 ε, CD4, CD5, CD8, CD21, CD22, CD28, CD32, CD40L (CD154), CD45, CD66d, CD79a, CD79b, CD80, CD86, CD278 (also known as ICOS), CD247 ζ, CD247 η, DAP10, DAP12, FYN, LAT, Lck, MAPK, MHC complex, NFAT, NF-κB, PLC-γ, iC3b, C3dg, C3d, and Zap70. In some embodiments, the signaling domain includes an immunoreceptor tyrosine-based activation motif or ITAM. A signaling domain comprising an ITAM can comprise two repeats of the amino acid sequence YxxL/I separated by 6-8 amino acids, wherein each x is independently any amino acid, producing the conserved motif YxxL/Ix(6-8)YxxL/I. A signaling domain comprising an ITAM can be modified, for example, by phosphorylation when the antigen binding domain is bound to an epitope. A phosphorylated ITAM can function as a docking site for other proteins, for example proteins involved in various signaling pathways. In some embodiments, the primary signaling domain comprises a modified ITAM domain, e.g., a mutated, truncated, and/or optimized ITAM domain, which has altered (e.g., increased or decreased) activity compared to the native ITAM domain.

In some embodiments, the intracellular signaling domain of a subject CAR (including aBA-CAR) comprises an FcyR signaling domain (e.g., ITAM). The FcyR signaling domain can be selected from FcyRI (CD64), FcγRIIA (CD32), FcγRIIB (CD32), FcγRIIIA (CD16a), and FcγRIIIB (CD16b). In some embodiments, the intracellular signaling domain comprises an FcεR signaling domain (e.g., ITAM). The FcεR signaling domain can be selected from FcεRI and FcεRII (CD23). In some embodiments, the intracellular signaling domain comprises an FcaR signaling domain (e.g., ITAM). The FcaR signaling domain can be selected from FcaRI (CD89) and Fcα/µR. In some embodiments, the intracellular signaling domain comprises a CD3 ζ signaling domain. In some embodiments, the primary signaling domain comprises an ITAM of CD3 ζ.

In some embodiments, an intracellular signaling domain of a subject CAR (including aBA-CAR) comprises an immunoreceptor tyrosine-based inhibition motif or ITIM. A signaling domain comprising an ITIM can comprise a conserved sequence of amino acids (S/I/V/LxYxxI/V/L) that is found in the cytoplasmic tails of some inhibitory receptors of the immune system. A primary signaling domain comprising an ITIM can be modified, for example phosphorylated, by enzymes (such as a Src kinase family member (e.g., Lck)). Following phosphorylation, other proteins, including enzymes, can be recruited to the ITIM. These other proteins include, but are not limited to, enzymes such as the phosphotyrosine phosphatases SHP-1 and SHP-2, the inositol-phosphatase called SHIP, and proteins having one or more SH2 domains (e.g., ZAP70). A intracellular signaling domain can comprise a signaling domain (e.g., ITIM) of BTLA, CD5, CD31, CD66a, CD72, CMRF35H, DCIR, EPO-R, FcγRIIB (CD32), Fc receptor-like protein 2 (FCRL2), Fc receptor-like protein 3 (FCRL3), Fc receptor-like protein 4 (FCRL4), Fc receptor-like protein 5 (FCRL5), Fc receptor-like protein 6 (FCRL6), protein G6b (G6B), interleukin 4 receptor (IL4R), immunoglobulin superfamily receptor translocation-associated 1 (IRTA1), immunoglobulin superfamily receptor translocation-associated 2 (IRTA2), killer cell immunoglobulin-like receptor 2DL1 (KIR2DL1), killer cell immunoglobulin-like receptor 2DL2 (KIR2DL2), killer cell immunoglobulin-like receptor 2DL3 (KIR2DL3), killer cell immunoglobulin-like receptor 2DL4 (KIR2DL4), killer cell immunoglobulin-like receptor 2DL5 (KIR2DL5), killer cell immunoglobulin-like receptor 3DL1 (KIR3DL1), killer cell immunoglobulin-like receptor 3DL2 (KIR3DL2), leukocyte immunoglobulin-like receptor subfamily B member 1 (LIR1), leukocyte immunoglobulin-like receptor subfamily B member 2 (LIR2), leukocyte immunoglobulin-like receptor subfamily B member 3 (LIR3), leukocyte immunoglobulin-like receptor subfamily B member 5 (LIR5), leukocyte immunoglobulin-like receptor subfamily B member 8 (LIR8), leukocyte-associated immunoglobulin-like receptor 1 (LAIR-1), mast cell function-associated antigen (MAFA), NKG2A, natural cytotoxicity triggering receptor 2 (NKp44), NTB-A, programmed cell death protein 1 (PD-1), PILR, SIGLECL1, sialic acid binding Ig like lectin 2 (SIGLEC2 or CD22), sialic acid binding Ig like lectin 3 (SIGLEC3 or CD33), sialic acid binding Ig like lectin 5 (SIGLEC5 or CD 170), sialic acid binding Ig like lectin 6 (SIGLEC6), sialic acid binding Ig like lectin 7 (SIGLEC7), sialic acid binding Ig like lectin 10 (SIGLEC10), sialic acid binding Ig like lectin 11 (SIGLEC11), sialic acid binding Ig like lectin 4 (SIGLEC4), sialic acid binding Ig like lectin 8 (SIGLEC8), sialic acid binding Ig like lectin 9 (SIGLEC9), platelet and endothelial cell adhesion molecule 1 (PECAM-1), signal regulatory protein (SIRP 2), and signaling threshold regulating transmembrane adaptor 1 (SIT). In some embodiments, the intracellular signaling domain comprises a modified ITIM domain, e.g., a mutated, truncated, and/or optimized ITIM domain, which has altered (e.g., increased or decreased) activity compared to the native ITIM domain.

In some embodiments, the intracellular signaling domain of CAR (including aBA-CAR) comprises at least 2 ITAM domains (e.g., at least 3, 4, 5, 6, 7, 8, 9, or 10 ITAM domains). In some embodiments, the intracellular signaling domain comprises at least 2 ITIM domains (e.g., at least 3, 4, 5, 6, 7, 8, 9, or 10 ITIM domains) (e.g., at least 2 primary signaling domains). In some embodiments, the intracellular signaling domain comprises both ITAM and ITIM domains.

In some cases, the intracellular signaling domain of CAR (inducing aBA-CAR) can include a co-stimulatory domain. In some embodiments, a co-stimulatory domain, for example from co-stimulatory molecule, can provide co-stimulatory signals for immune cell signaling, such as signaling from ITAM and/or ITIM domains, e.g., for the activation and/or deactivation of immune cell activity. In some embodiments, a co-stimulatory domain is operable to regulate a proliferative and/or survival signal in the immune cell. In some embodiments, a co-stimulatory signaling domain comprises a signaling domain of a MHC class I protein, MHC class II protein, TNF receptor protein, immunoglobulin-like protein, cytokine receptor, integrin, signaling lymphocytic activation molecule (SLAM protein), activating NK cell receptor, BTLA, or a Toll ligand receptor. In some embodiments, the co-stimulatory domain comprises a signaling domain of a molecule selected from the group consisting of: 2B4/CD244/SLAMF4, 4-1BB/TNFSF9/CD137, B7-1/CD80, B7-2/CD86, B7-H1/PD-L1, B7-H2, B7-H3, B7-H4, B7-H6, B7-H7, BAFF R/TNFRSF13C, BAFF/BLyS/TNFSF13B, BLAME/SLAMF8, BTLA/CD272, CD100 (SEMA4D), CD103, CD11a, CD11b, CD11c, CD11d, CD150, CD160 (BY55), CD18, CD19, CD2, CD200, CD229/SLAMF3, CD27 Ligand/TNFSF7, CD27/TNFRSF7, CD28, CD29, CD2F-10/SLAMF9, CD30 Ligand/TNFSF8, CD30/TNFRSF8, CD300a/LMIR1, CD4, CD40 Ligand/TNFSF5, CD40/TNFRSF5, CD48/SLAMF2, CD49a, CD49D, CD49f, CD5, CD53, CD58/LFA-3, CD69, CD7, CD8 α, CD8 β, CD82/Kai-1, CD84/SLAMF5, CD90/Thy1, CD96, CDS, CEACAM1, CRACC/SLAMF7, CRTAM, CTLA-4, DAP12, Dectin-1/CLEC7A, DNAM1 (CD226), DPPIV/CD26, DR3/TNFRSF25, EphB6, GADS, Gi24/VISTA/B7-H5, GITR Ligand/TNFSF18, GITR/TNFRSF18, HLA Class I, HLA-DR, HVEM/TNFRSF14, IA4, ICAM-1, ICOS/CD278, Ikaros, IL2R β, IL2R γ, IL7R α, Integrin α4/CD49d, Integrin α4β1, Integrin α4p7/LPAM-1, IPO-3, ITGA4, ITGA6, ITGAD, ITGAE, ITGAL, ITGAM, ITGAX, ITGB1, ITGB2, ITGB7, KIRDS2, LAG-3, LAT, LIGHT/TNFSF14, LTBR, Ly108, Ly9 (CD229), lymphocyte function associated antigen-1 (LFA-1), Lymphotoxin-α/TNF-β, NKG2C, NKG2D, NKp30, NKp44, NKp46, NKp80 (KLRF1), NTB-A/SLAMF6, OX40 Ligand/TNFSF4, OX40/TNFRSF4, PAG/Cbp, PD-1, PDCD6, PD-L2/B7-DC, PSGL1, RELT/TNFRSF19L, SELPLG (CD162), SLAM (SLAMF1), SLAM/CD150, SLAMF4 (CD244), SLAMF6 (NTB-A), SLAMF7, SLP-76, TACI/TNFRSF13B, TCL1A, TCL1B, TIM-1/KIM-1/HAVCR, TIM-4, TL1A/TNFSF15, TNF RII/TNFRSF1B, TNF-α, TRANCE/RANKL, TSLP, TSLP R, VLA1, and VLA-6. In some embodiments, the intracellular signaling domain comprises multiple co-stimulatory domains, for example at least two, e.g., at least 3, 4, or 5 co-stimulatory domains. Co-stimulatory signaling regions may provide a signal synergistic with the primary effector activation signal and can complete the requirements for activation of a T cell. In some embodiments, the addition of co-stimulatory domains to the CAR can enhance the efficacy and persistence of the immune cells provided herein.

Binding of the CAR, in particular aBA-CAR, to the booster antigen can enhance immune cell proliferation as compared to an immune cell lacking the CAR, in particular aBA-CAR. Proliferation of the immune cell can refer to expansion of the immune cell. Proliferation of the immune cell can refer to phenotypic changes of the immune cell. Proliferation of an immune cell comprising a CAR in particular aBA-CAR provided herein can be greater than that of a comparable immune cell lacking the CAR. Proliferation of an immune cell comprising the CAR can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, from about 200 fold to about 300 fold, from about 300 fold to about 400 fold, from about 400 fold to about 500 fold, from about 500 fold to about 600 fold, from about 600 fold to about 700 fold greater than the proliferation of a comparable immune cell lacking the CAR. Proliferation of an immune cell comprising the CAR can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, from about 200 fold to about 300 fold, from about 300 fold to about 400 fold, from about 400 fold to about 500 fold, from about 500 fold to about 600 fold, from about 600 fold to about 700 fold greater than the proliferation of a comparable immune cell lacking the CAR, and wherein the proliferation is ascertained at least about 12, 24, 36, 48, 60, 72, 84, or 96 hours after contacting the booster antigen to the immune cell. The enhanced proliferation can be ascertained either *in vitro* or *in vivo.* In some embodiments, proliferation can comprise quantifying the number of immune cells. Quantifying a number of immune cells can comprise flow cytometry, Trypan Blue exclusion, and/or hemocytometry. Proliferation can also be determined by phenotypic analysis of the immune cells.

In some cases, the CAR structure may include an expression control element, which may be a switch element that regulates the expression of CAR or CAR-T cells and/or makes CAR-T or CAR-NK cells committed to suicide or apoptosis, such as TET-ON.

In some embodiments, the therapeutic immune cells are T cells, or NK cells.

In some embodiments, the therapeutic immune cells are artificially modified T cells, or NK cells, such as CAR-T, CAR-NK, TCR-T, or TCR-NK cells.

In an aspect, the therapeutic immune of the present disclosure is tumor infiltrating lymphocyte (TIL) that specifically binds to tumor associated antigen, including but not limited to a neoantigen. Modified TIL can comprise a chimeric stimulating molecule. The chimeric stimulating molecule can comprise a polypeptide extracellular domain (PED) that binds to the neoantigen. The PED can be fused to an intracellular domain (ICD) of a co-stimulatory molecule that mediates an immune cell activation signal. Binding of the chimeric stimulating molecule to the neoantigen can yield the immune cell activation signal in the modified TIL. In some embodiments, the PED can be an extracellular domain of a surface protein of an unmodified TIL. In some embodiments, examples of the PED include antibodies, as well as derivatives, variants, and fragments thereof.

In one aspect, the present disclosure provides modified T cells that have dual recognition properties, with one aspect of the dual recognition referring to the recognition of target cells, that is, the T cell on one hand can identify and kill target cells, which are cells harmful to human body, such as tumor cells, or non-tumor cells such as viruses and bacteria; another aspect referring to the recognition of booster antigen, that is, the immune cells can independently recognize the booster antigen or an adjuvant having a booster antigen and can be activated to expand its quantity.

In one aspect, the present disclosure provides T cells modified with aBA-CAR, wherein the T cells have dual recognition properties, with one aspect of the dual recognition referring to the recognition of target cells, that is, the T cell on one hand can identify and kill target cells, which are cells harmful to human body, such as tumor cells, or non-tumor cells such as viruses and bacteria; another aspect referring to the recognition of booster antigen, that is, the immune cells can independently recognize the booster antigen or an adjuvant having a booster antigen via aBA-CAR and can be activated to expand its quantity.

In one aspect, the present disclosure provides T cells modified with aBA-CAR along with TCR or CAR (another CAR different from aBA-CAR), wherein the T cells have dual recognition properties, with one aspect of the dual recognition referring to the recognition of target cells, that is, the T cells with artificial (or referred as heterologous) TCR or CAR (another CAR different from aBA-CAR) can identify and kill target cells via the TCR or CAR, wherein said target cells are harmful to human body, such as tumor cells, or non-tumor cells such as viruses and bacteria; another aspect referring to the recognition of booster antigen, that is, the immune cells can independently recognize the booster antigen or an adjuvant having a booster antigen via aBA-CAR and can be activated to expand its quantity.

In some embodiments, the target of target cell recognized by the immune cell and the target of the booster antigen are different or the same.

In one aspect, the present disclosure provides tumor-recognizing T cells or tumor antigen-reactive T cells modified with aBA-CAR, wherein the T cells have dual recognition properties, with one aspect of the dual recognition referring to the recognition of rumor cells, that is, the T cells on one hand can identify and kill tumor cells; another aspect referring to the recognition of booster antigen, that is, the immune cells can independently recognize the booster antigen or an adjuvant having a booster antigen via aBA-CAR and can be activated to expand its quantity.

The recognition of tumor by tumor-recognizing T cells (or tumor antigen-reactive T cells) can be recognition of neoantigens (neoantigen), tumor-associated antigens (TAA), or cancer testis antigens, viral antigens (such as viral antigens on the surface of cells which are infected with HPV or EBV virus and become cancerous) or the like via unmodified natural TCR. Such tumor-recognizing T cells can be obtained by vaccine induction, or they can be obtained from tumor infiltrating lymphocytes (TIL), or they can be obtained by positive or negative screening with certain markers of peripheral blood (such as PD1, TIM3, CD137, CD39, CD28, etc.).

The recognition of tumors by the tumor-recognizing T cells can also be by means of artificially modified CAR or TCR and formation of CAR-T or TCR-T to recognize corresponding tumor cells.

The tumor infiltrating lymphocyte (TIL) can be any cell obtained from a tumor. For example, the TIL can be a cell that has migrated to a tumor. A TIL can be a cell that has infiltrated a tumor. In some embodiments, the TIL is a white blood cell that has migrated into a tumor from the bloodstream of a subject. A TIL can be, for example, a T cell, B cell, monocyte, or natural killer (NK) cell. In some cases, a modified TIL comprises a CD8+ cytotoxic T cell (lymphocyte), Th1 and Th17 CD4+ T cell, a natural killer cell, a dendritic cell, or M1 macrophage. A population of immune cells comprising TILs can be a mixed population of cells. A population of TILs can comprise cells of different phenotypes, cells of different degrees of differentiation, cells of different lineages, or any combination thereof. TILs can generally be defined either biochemically, using cell surface markers, or functionally, by their ability to infiltrate tumors and effect treatment. TILs can be categorized based on expression one or more of the following biomarkers: CD4, CD8, TCR alpha beta, CD25, CD27, CD28, CD56, CD137, CCR7, CD45Ra, CD95, PD-1, and TIM-3. In some embodiments, the modified TIL expresses at least one of PD-1, CD137, and TIM-3. In some cases, a TIL can be functionally defined by its ability to infiltrate solid tumors upon reintroduction into a patient. In some cases, the modified TIL comprises a "primary TIL," referring to a TIL that is obtained from a patient tissue sample. In some cases, the modified TIL comprises a "secondary TILs," referring to a TIL that is has been expanded or proliferated. A TIL can exhibit specific binding to a neoantigen. In some cases, the TCR complex of the TIL confers the antigen binding specificity (e.g., neoantigen binding).

In some embodiments, the immune cell is a tumor infiltrating lymphocyte (TIL). The TIL can be, for example, a T cell, B cell, monocyte, or natural killer (NK) cell. In some cases, the TIL comprises a CD8+ cytotoxic T cell (lymphocyte), Th1 and Th17 CD4+ T cell, a natural killer cell, a dendritic cell, or M1 macrophage. In some embodiments, the TIL can express at least one of PD-1, CD137, and TIM-3. In some cases, the modified TIL comprises a "primary TIL," referring to a TIL that is obtained from a patient tissue sample. In some cases, the modified TIL comprises a "secondary TILs," referring to a TIL that is has been expanded or proliferated.

In some cases, TIL may escape from tumor tissue and exist in peripheral blood. In some embodiments, TIL may not be derived from tumor tissue. In some embodiments, TIL can be obtained by isolating peripheral blood mononuclear cells (PBMC) by apheresis, or by further screening or enrichment conducted with one or more markers such as PD1, TIM3, CD137, and CD39.

In an aspect, the present disclosure provides a modified T cell that specifically binds to a neoantigen, the modified T cell not only comprising aBA-CAR, but also an enhanced receptor. The enhanced receptor may comprise an extracellular domain (ECD) of a protein. The ECD can be fused to an intracellular domain (ICD) of a co-stimulatory molecule that mediates an immune cell activation signal. Binding of the enhanced receptor to the ligand may yield the immune cell activation signal in the modified T cell instead of the immune cell inactivation signal.

The modified T cell can comprise a T cell receptor (TCR) complex which exhibits specific binding to the neoantigen. In some embodiments, the TCR complex is an endogenous TCR complex. In some embodiments, the TCR is an exogenous TCR complex. The TCR complex, e.g., endogenous or exogenous, of the modified immune cell can confer the antigen binding specificity (e.g., neoantigen binding) of the immune cell. In some embodiments, the present disclosure provides a modified T cell comprising an endogenous TCR complex that specifically binds to a neoantigen, said modified T cell comprising a chimeric stimulating molecule, wherein said chimeric stimulating molecule comprises: a polypeptide extracellular domain (PED) that binds to a membrane protein on a cell including but not limited to a tumor cell, wherein said PED is fused to an intracellular domain (ICD) of a co-stimulatory molecule that mediates an immune cell activation signal, wherein binding of said chimeric stimulating molecule to said membrane protein yields said immune cell activation signal in said modified T cell.

Binding of a modified immune cell, such as a modified T cell or a modified TIL provided herein, to a neoantigen can activate the immune cell. The enhanced receptor of the modified cell can be used to provide further control over immune cell activities, such as but not limited to, immune cell activation and expansion. Binding of the enhanced receptor to its ligand in the modified immune cell, such as a modified T cell or modified TIL, can elicit an immune cell activation signal in the modified immune cell instead of the immune cell inactivation signal. Eliciting the immune cell activation signal in the modified immune cell instead of the immune cell inactivation signal may minimize an immune-suppressive effect in the immune cell. Minimizing an immune-suppressive effect in the immune cell can increase the effectiveness of the immune cell in an immune response, for example by increasing immune cell cytotoxicity against a target cell, such as a tumor cell.

The enhanced receptor can comprise an extracellular domain (ECD) of a protein that, in an unmodified immune cell, elicits an immune cell signal, which can be either an inactivation signal or an activation signal, or neither an inactivation signal nor an activation signal, upon binding to its ligand. The protein can be a signaling receptor or any functional fragment, derivative, or variant thereof. In some cases, the signaling receptor can be a membrane bound receptor. A signaling receptor can, in response to ligand binding, induce one or more signaling pathways in a cell. In some cases, the signaling receptor can be a non-membrane bound receptor. The enhancer receptor can comprise a fragment, for example an extracellular domain, of a receptor selected from a G-protein coupled receptor (GPCR); an integrin receptor; a cadherin receptor; a catalytic receptor (e.g., kinases); a death receptor; a checkpoint receptor; a cytokine receptor; a chemokine receptor; a growth factor receptor; a hormone receptor; and an immune receptor.

In some embodiments, the enhancer receptor comprises a fragment of an immune checkpoint receptor, which may be involved in regulation of the immune system. Non-limiting examples of such receptors include, but are not limited to, programmed cell death 1 (PD-1), cytotoxic T-lymphocyte associated protein 4 (CTLA-4), B and T lymphocyte attenuator (BTLA), a killer immunoglobulin-like receptor (KIR), indoleamine 2,3-dioxygenase (IDO), lymphocyte activation gene-3 (LAG3), T cell immunoglobulin mucin 3 (TIM-3), T-cell immunoreceptor with Ig and ITIM domains (TIGIT), SIRPα and NKG2D.

In some embodiments, the enhancer receptor comprises at least an extracellular fragment of a TCR, which may be involved in recognizing a neoantigen of a target cell (e.g., a cancer cell antigen or a tumor antigen). In some examples, the enhancer receptor can comprise the extracellular variable regions of the TCR α and/or β chains.

An enhancer receptor can comprise an immune checkpoint receptor, or any derivative, variant or fragment thereof. An enhancer receptor can bind an antigen comprising any suitable immune checkpoint receptor ligand, or any derivative, variant or fragment thereof. Non-limiting examples of such ligands include, but are not limited to, B7-1, B7-H3, B7-H4, HVEM (Herpesvirus Entry Mediator), AP2M1, CD80, CD86, SHP-2, PPP2R5A, MHC (e.g., class I, class II), CD47, CD70, PD-L1 (or PDL1), and PD-L2. The region of an enhancer receptor which binds to such ligands can be a natural receptor of such ligands or a monoclonal antibody of such ligands.

In some embodiments, the enhancer receptor comprises a fragment of a cytokine receptor. Cytokine receptors can serve a variety of functions, non-limiting examples of which include immune cell regulation and mediating inflammation. In some embodiments, the enhancer receptor comprises a cytokine receptor, for example a type I cytokine receptor or a type II cytokine receptor, or any derivative, variant or fragment thereof. In some embodiments, the enhancer receptor comprises an interleukin receptor (e.g., IL-2R, IL-3R, IL-4R, IL-5R, IL-6R, IL-7R, IL-9R, IL-11R, IL-12R, IL-13R, IL-15R, IL-21R, IL-23R, IL-27R, and IL-31R), a colony stimulating factor receptor (e.g., erythropoietin receptor, CSF-1R, CSF-2R, GM-CSFR, and G-CSFR), a hormone receptor/neuropeptide receptor (e.g., growth hormone receptor, prolactin receptor, and leptin receptor), or any derivative, variant or fragment thereof. In some embodiments, the enhancer receptor comprises a type II cytokine receptor, or any derivative, variant or fragment thereof. In some embodiments, the enhancer receptor comprises an interferon receptor (e.g., IFNAR1, IFNAR2, and IFNGR), an interleukin receptor (e.g., IL-10R, IL-20R, IL-22R, and IL-28R), a tissue factor receptor (also called platelet tissue factor), or any derivative, variant or fragment thereof.

In some embodiments, the extracellular region of enhancer receptor can be any antibody or antibody fragment, the antigen to which said antibody binds can be any membrane protein widely expressed by human cells or a membrane protein expressed by tumor cells, wherein these membrane proteins include a class I RTK (e.g., the epidermal growth factor (EGF) receptor family including EGFR; the ErbB family including ErbB-2, ErbB-3, and ErbB-4), a class II RTK (e.g., the insulin receptor family including INSR, IGF-1R, and IRR), a class III RTK (e.g., the platelet-derived growth factor (PDGF) receptor family including PDGFR-α, PDGFR-β, CSF-1R, KIT/SCFR, and FLK2/FLT3), a class IV RTK (e.g., the fibroblast growth factor (FGF) receptor family including FGFR-1, FGFR-2, FGFR-3, and FGFR-4), a class V RTK (e.g., the vascular endothelial growth factor (VEGF) receptor family including VEGFR1, VEGFR2, and VEGFR3), a class VI RTK (e.g., the hepatocyte growth factor (HGF) receptor family including hepatocyte growth factor receptor (HGFR/MET) and RON), a class VII RTK (e.g., the tropomyosin receptor kinase (Trk) receptor family including TRKA, TRKB, and TRKC), a class VIII RTK (e.g., the ephrin (Eph) receptor family including EPHA1, EPHA2, EPHA3, EPHA4, EPHA5, EPHA6, EPHA7, EPHA8, EPHB1, EPHB2, EPHB3, EPHB4, EPHB5, and EPHB6), a class IX RTK (e.g., AXL receptor family such as AXL, MER, and TRYO3), a class X RTK (e.g., LTK receptor family such as LTK and ALK), a class XI RTK (e.g., TIE receptor family such as TIE and TEK), a class XII RTK (e.g., ROR receptor family ROR1 and ROR2), a class XIII RTK (e.g., the discoidin domain receptor (DDR) family such as DDR1 and DDR2), a class XIV RTK (e.g., RET receptor family such as RET), a class XV RTK (e.g., KLG receptor family including PTK7), a class XVI RTK (e.g., RYK receptor family including Ryk), a class XVII RTK (e.g., MuSK receptor family such as MuSK), CD47, CD70, NKG2D, or any derivative, variant or fragment thereof.

In some embodiments, the membrane protein to which the enhancer receptor can bind may comprise at least an extracellular region (e.g., ligand binding domain) of a catalytic receptor (such as a receptor tyrosine kinase (RTK)), or any derivative, variant or fragment thereof, or comprise a fragment of variable region of an antibody with such fragment as antigen. In some embodiments, the membrane protein which can be bound by the enhancer receptor comprises a class I RTK (e.g., the epidermal growth factor (EGF) receptor family including EGFR; the ErbB family including ErbB-2, ErbB-3, and ErbB-4), a class II RTK (e.g., the insulin receptor family including INSR, IGF-1R, and IRR), a class III RTK (e.g., the platelet-derived growth factor (PDGF) receptor family including PDGFR-α, PDGFR-β, CSF-1R, KIT/SCFR, and FLK2/FLT3), a class IV RTK (e.g., the fibroblast growth factor (FGF) receptor family including FGFR-1, FGFR-2, FGFR-3, and FGFR-4), a class V RTK (e.g., the vascular endothelial growth factor (VEGF) receptor family including VEGFR1, VEGFR2, and VEGFR3), a class VI RTK (e.g., the hepatocyte growth factor (HGF) receptor family including hepatocyte growth factor receptor (HGFR/MET) and RON), a class VII RTK (e.g., the tropomyosin receptor kinase (Trk) receptor family including TRKA, TRKB, and TRKC), a class VIII RTK (e.g., the ephrin (Eph) receptor family including EPHA1, EPHA2, EPHA3, EPHA4, EPHA5, EPHA6, EPHA7, EPHA8, EPHB1, EPHB2, EPHB3, EPHB4, EPHB5, and EPHB6), a class IX RTK (e.g., AXL receptor family such as AXL, MER, and TRYO3), a class X RTK (e.g., LTK receptor family such as LTK and ALK), a class XI RTK (e.g., TIE receptor family such as TIE and TEK), a class XII RTK (e.g., ROR receptor family ROR1 and ROR2), a class XIII RTK (e.g., the discoidin domain receptor (DDR) family such as DDR1 and DDR2), a class XIV RTK (e.g., RET receptor family such as RET), a class XV RTK (e.g., KLG receptor family including PTK7), a class XVI RTK (e.g., RYK receptor family including Ryk), a class XVII RTK (e.g., MuSK receptor family such as MuSK), CD47, CD70, NKG2D, or any derivative, variant or fragment thereof.

In some embodiments, the membrane protein to which the enhancer receptor can bind comprise a RTK, or any derivative, variant or fragment thereof, can bind an antigen comprising any suitable RTK ligand, or any derivative, variant or fragment thereof, or comprise a fragment of variable region of an antibody with such fragment as antigen. Non limiting examples of RTK ligands include growth factors, cytokines, and hormones. Growth factors include, for example, members of the epidermal growth factor family (e.g., epidermal growth factor or EGF, heparin-binding EGF-like growth factor or HB-EGF, transforming growth factor-a or TGF-α, amphiregulin or AR, epiregulin or EPR, epigen, betacellulin or BTC, neuregulin-1 or NRG1, neuregulin-2 or NRG2, neuregulin-3 or NRG3, and neuregulin-4 or NRG4), the fibroblast growth factor family (e.g., FGF1, FGF2, FGF3, FGF4, FGF5, FGF6, FGF7, FGF8, FGF9, FGF10, FGF11, FGF12, FGF13, FGF14, FGF15/19, FGF16, FGF17, FGF18, FGF20, FGF21, and FGF23), the vascular endothelial growth factor family (e.g., VEGF-A, VEGF-B, VEGF-C, VEGF-D, and PIGF), and the platelet-derived growth factor family (e.g., PDGFA, PDGFB, PDGFC, and PDGFD). Hormones include, for example, members of the insulin/IGF/relaxin family (e.g., insulin, insulin-like growth factors, relaxin family peptides including relaxin1, relaxin2, relaxin3, Leydig cell-specific insulin-like peptide (gene INSL3), early placenta insulin-like peptide (ELIP) (gene INSL4), insulin-like peptide 5 (gene INSL5), and insulin-like peptide 6).

In some embodiments, the membrane protein to which the enhancer receptor can bind comprises at least an extracellular region (e.g., ligand binding domain) of a catalytic receptor such as a receptor threonine/serine kinase (RTSK), or any derivative, variant or fragment thereof, or comprise a fragment of variable region of an antibody with such fragment as antigen. The membrane protein to which the enhancer receptor can bind can comprise a type I RTSK, type II RTSK, or any derivative, variant or fragment thereof. The enhancer receptor can comprise a type I receptor, or any derivative, variant or fragment thereof, selected from the group consisting of: ALK1 (ACVRL1), ALK2 (ACVR1A), ALK3 (BMPR1A), ALK4 (ACVR1B), ALK5 (TGFβR1), ALK6 (BMPR1B), and ALK7 (ACVR1C). An enhancer receptor can comprise a type II receptor, or any derivative, variant or fragment thereof, selected from the group consisting of: TGFβR2, BMPR2, ACVR2A, ACVR2B, and AMHR2 (AMHR). In some embodiments, the switch molecule comprises a TGF-β receptor, or any derivative, variant or fragment thereof.

A membrane protein, to which the enhancer receptor can bind, comprising a RTSK, or any derivative, variant or fragment thereof, can bind an antigen comprising any suitable RTSK ligand, or any derivative, variant or fragment thereof, or comprise a fragment of variable region of an antibody with such fragment as antigen.

The enhancer receptor can comprise an intracellular domain (ICD) of a co-stimulatory molecule that elicits an immune cell activation signal. The co-stimulatory molecule may bind a ligand. In some cases, the co-stimulatory molecule may be activated by a ligand responsive protein. In some embodiments, the co-stimulatory molecule is operable to regulate a proliferative and/or survival signal in the immune cell. In some embodiments, the ICD is an intracellular domain of a co-stimulatory molecule selected from an MHC class I protein, an MHC class II protein, a TNF receptor protein, an immunoglobulin-like protein, a cytokine receptor, an integrin, a signaling lymphocytic activation molecule (SLAM protein), an activating NK cell receptor, BTLA, or a Toll ligand receptor. In some embodiments, the co-stimulatory domain comprises a signaling domain of a molecule selected from the group consisting of: 2B4/CD244/SLAMF4, 4-1BB/TNFSF9/CD137, B7-1/CD80, B7-2/CD86, B7-H1/PD-L1, B7-H2, B7-H3, B7-H4, B7-H6, B7-H7, BAFF R/TNFRSF13C, BAFF/BLyS/TNFSF13B, BLAME/SLAMF8, BTLA/CD272, CD100 (SEMA4D), CD103, CD11a, CD11b, CD11c, CD11d, CD150, CD160 (BY55), CD18, CD19, CD2, CD200, CD229/SLAMF3, CD27 Ligand/TNFSF7, CD27/TNFRSF7, CD28, CD29, CD2F-10/SLAMF9, CD3, CD30 Ligand/TNFSF8, CD30/TNFRSF8, CD300a/LMIR1, CD4, CD40 Ligand/TNFSF5, CD40/TNFRSF5, CD48/SLAMF2, CD49a, CD49D, CD49f, CD5, CD53, CD58/LFA-3, CD69, CD7, CD8 α, CD8 β, CD82/Kai-1, CD84/SLAMF5, CD90/Thy1, CD96, CDS, CEACAM1, CRACC/SLAMF7, CRTAM, CTLA-4, DAP12, Dectin-1/CLEC7A, DNAM1 (CD226), DPPIV/CD26, DR3/TNFRSF25, EphB6, GADS, Gi24/VISTA/B7-H5, GITR Ligand/TNFSF18, GITR/TNFRSF18, HLA Class I, HLA-DR, HVEM/TNFRSF14, IA4, ICAM-1, ICOS/CD278, Ikaros, IL2R β, IL2R γ, IL7R α, IL-12R, Integrin α4/CD49d, Integrin α4β1, Integrin α4β7/LPAM-1, IPO-3, ITGA4, ITGA6, ITGAD, ITGAE, ITGAL, ITGAM, ITGAX, ITGB1, ITGB2, ITGB7, KIRDS2, LAG-3, LAT, LIGHT/TNFSF14, LTBR, Ly108, Ly9 (CD229), lymphocyte function associated antigen-1 (LFA-1), Lymphotoxin-α/TNF-β, NKG2C, NKG2D, NKp30, NKp44, NKp46, NKp80 (KLRF1), NTB-A/SLAMF6, 0X40 Ligand/TNFSF4, OX40/TNFRSF4, PAG/Cbp, PD-1, PDCD6, PD-L2/B7-DC, PSGL1, RELT/TNFRSF19L, SELPLG (CD162), SLAM (SLAMF1), SLAM/CD150, SLAMF4 (CD244), SLAMF6 (NTB-A), SLAMF7, SLP-76, TACI/TNFRSF13B, TCL1A, TCL1B, TIM-1/KIM-1/HAVCR, TIM-4, TL1A/TNFSF15, TNF RII/TNFRSF1B, TNF-a, TRANCE/RANKL, TSLP, TSLP R, VLA1, and VLA-6.

The ECD and the ICD of an enhancer receptor can be joined by a transmembrane domain, for example by a membrane spanning segment. In some embodiments, the membrane spanning segment comprises a polypeptide. The membrane spanning polypeptide can have any suitable polypeptide sequence. In some cases, the membrane spanning polypeptide comprises a polypeptide sequence of a membrane spanning portion of an endogenous or wild-type membrane spanning protein. In some embodiments, the membrane spanning polypeptide comprises a polypeptide sequence having at least 1 (e.g., at least 2, 3, 4, 5, 6, 7, 8, 9, 10 or greater) of an amino acid substitution, deletion, and insertion compared to a membrane spanning portion of an endogenous or wild-type membrane spanning protein. In some embodiments, the membrane spanning polypeptide comprises a non-natural polypeptide sequence, such as the sequence of a polypeptide linker. The polypeptide linker may be flexible or rigid. The polypeptide linker can be structured or unstructured. In some embodiments, the membrane spanning polypeptide transmits a signal from the ECD to the ICD, for example a signal indicating ligand-binding. In some embodiments, ECD comprises the transmembrane domain. In some embodiments, ICD comprises the transmembrane domain.

In some embodiments, ICD can mediate and yield an immune cell activation signal in the immune cell. In some embodiments, the immune cell activation signal is mediated by an activation factor. The activation factor can be an immunomodulating molecule. The activation factor may bind, activate, or stimulate T cells or other immune cells to modulate their activity. In some embodiments, the activation factor can be secreted from the immune cell. The activation factor can be, for example, a soluble cytokine, a soluble chemokine, or a growth factor molecule. Non-limiting examples of activation factors which can mediate the immune cell activation include a soluble cytokine, such as IL-1, IL-2, IL-6, IL-7, IL-8, IL-10, IL-12, IL-15, IL-21, tumor necrosis factor (TNF), transforming growth factor (TGF), interferon (IFN), or any functional fragment or variant thereof.

The immune cell activation signal can comprise or result in a clonal expansion of the modified immune cell (e.g., modified TIL or modified T cell); cytokine release by the modified immune cell (e.g., modified TIL or modified T cell); cytotoxicity of the modified immune cell (e.g., modified TIL or modified T cell); proliferation of the modified immune cell (e.g., modified TIL or modified T cell); differentiation, dedifferentiation or transdifferentiation of the modified immune cell (e.g., modified TIL or modified T cell); movement and/or trafficking of the modified immune cell (e.g., modified TIL or modified T cell); exhaustion and/or reactivation of the modified immune cell (e.g., modified TIL or modified T cell); and release of other intercellular molecules, metabolites, chemical compounds, or combinations thereof by the modified immune cell (e.g., modified TIL or modified T cell).

In some embodiments, the immune cell activation signal comprises or results in clonal expansion of the immune cell. Clonal expansion can comprise the generation of daughter cells arising from the immune cell. The daughter cells resulting from clonal expansion can comprise the enhancer receptor. Clonal expansion of the modified immune cell can be greater than that of a comparable immune cell lacking the enhancer receptor. Clonal expansion of the modified immune cell can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, about 200 fold to about 300 fold, about 300 fold to about 400 fold, about 400 fold to about 500 fold, about 500 fold to about 600 fold, or about 600 fold to about 700 fold greater than a comparable immune cell lacking the enhancer receptor. In some embodiments, determining clonal expansion can comprise quantifying a number of immune cells, for example with and without enhancer receptors and after ligand binding to the enhancer receptor. Quantifying a number of immune cells can be achieved by a variety of techniques, non-limiting examples of which include flow cytometry, Trypan Blue exclusion, and hemocytometry.

In some embodiments, the immune cell activation signal comprises or results in cytokine release by the immune cell. In some embodiments, the immune cell activity comprises or results in the release of intercellular molecules, metabolites, chemical compounds or combinations thereof. Cytokine release by the modified immune cell can comprise the release of IL-1, IL-2, IL-4, IL-5, IL-6, IL-13, IL-17, IL-21, IL-22, IPNγ, TNFα, CSF, TGFβ granzyme, and the like. In some embodiments, cytokine release may be quantified using enzyme-linked immunosorbent assay (ELISA), flow cytometry, western blot, and the like. Cytokine release by a modified immune cell can be greater than that of a comparable immune cell lacking the enhancer receptor. A modified immune cell provided herein can generate about 1 fold, 2 fold, 3 fold, 4 fold, 5 fold, 6 fold, 7 fold, 8 fold, 9 fold, 10 fold, 11 fold, 12 fold, 13 fold, 14 fold, 15 fold, 20 fold, 30 fold, 40 fold, 50 fold, 60 fold, 70 fold, 80 fold, 90 fold, 100 fold, 150 fold, 200 fold, 250 fold, or over 300 fold greater cytokine release as compared to a comparable immune cell lacking the enhancer receptor. The modified immune cell can exhibit increased cytokine secretion as compared to a comparable immune cell lacking the enhancer receptor (e.g., unmodified), when the enhancer receptor binds to the ligand and the modified immune cell binds to the neoantigen present on a target cell. In some embodiments, the cytokine secreted is IPNγ or IL-2. In some embodiments, cytokine release can be quantified *in vitro* or *in vivo.*

In some embodiments, the immune cell activation signal comprises or results in cytotoxicity of the immune cell. In some cases, cytotoxicity of the modified immune cells provided herein can be used for killing a target cell. An immune cell or population of immune cells expressing an enhancer receptor can induce death of a target cell. Killing of a target cell can be useful for a variety of applications, including, but not limited to, treating a disease or disorder in which a cell population is desired to be eliminated or its proliferation desired to be inhibited. Cytotoxicity can also refer to the release of cytotoxic cytokines, for example IPNγ or granzyme, by the immune cell. In some cases, modified immune cells provided herein may have altered (i) release of cytotoxins such as perforin, granzymes, and granulysin and/or (ii) induction of apoptosis via Fas-Fas ligand interaction between the T cells and target cells. In some embodiments, cytotoxicity can be quantified by a cytotoxicity assay including, a co-culture assay, ELISPOT, chromium release cytotoxicity assay, and the like. Cytotoxicity of a modified immune cell provided herein can be greater than that of a comparable immune cell lacking the enhancer receptor. The modified immune cell can exhibit increased cytotoxicity against a target cell as compared to a comparable immune cell lacking the enhancer receptor (e.g., unmodified), when the enhancer receptor binds to the ligand and the modified immune cell binds to the neoantigen present on the target cell. A modified immune cell of the present disclosure can be about 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, or 200% more cytotoxic to target cells as compared to a comparable immune cell lacking the enhancer receptor. A modified immune cell of the present disclosure can induce death of target cells that is at least 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, or 200% greater than that of a comparable immune cell lacking the modification. In some embodiments, an immune cell provided herein can induce apoptosis in target cells displaying target epitopes (e.g., neoantigens) on their surface. In some embodiments, cytotoxicity can be determined *in vitro* or *in vivo.* In some embodiments, determining cytotoxicity can comprise determining a level of disease after administration of modified immune cells provided herein as compared to a level of disease prior to the administration. In some embodiments, determining cytotoxicity can comprise determining a level of disease after administration of modified immune cells provided herein and a level of disease after administration of comparable immune cells lacking the enhancer receptor.

In some embodiments, immune cell activation signal comprises or results in proliferation of the immune cell. Proliferation of the immune cell can refer to expansion of the immune cell. Proliferation of the immune cell can refer to phenotypic changes of the immune cell. Proliferation of a modified immune cell of the present disclosure can be greater than that of a comparable immune cell lacking the enhancer receptor. Proliferation of a modified immune cell provided herein can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, from about 200 fold to about 300 fold, from about 300 fold to about 400 fold, from about 400 fold to about 500 fold, from about 500 fold to about 600 fold, or from about 600 fold to about 700 fold greater than the proliferation of a comparable immune cell lacking the enhancer receptor. In some embodiments, proliferation can be determined by quantifying a number of immune cells. Quantifying a number of immune cells can comprise flow cytometry, Trypan Blue exclusion, and/or hemocytometry. Proliferation can also be determined by phenotypic analysis of the immune cells.

In some embodiments, immune cell activation signal can comprise or result in differentiation, dedifferentiation, or transdifferentiation of the immune cell. Differentiation, dedifferentiation, or transdifferentation of an immune cell can be determined by evaluating phenotypic expression of markers of differentiation, dedifferentiation, or transdifferentation on a cell surface by flow cytometry. In some embodiments, a modified immune cell provided herein has increased differentiation ability as compared to a comparable immune cell lacking the enhancer receptor. In some embodiments, an modified immune cell provided herein has increased dedifferentiation ability as compared to a comparable immune cell lacking the enhancer receptor. In some embodiments, an modified immune cell provided herein has increased dedifferentiation ability as compared to a comparable immune cell lacking the enhancer receptor. In some embodiments, a modified immune cell provided herein has greater transdifferentiation ability as compared to a comparable immune cell lacking the enhancer receptor.

In some embodiments, immune cell activation signal can comprise or result in movement and/or trafficking of the immune cell. In some embodiments, movement can be determined by quantifying localization of the immune cell to a target site. For example, modified immune cells provided herein can be quantified at a target site after administration, for example at a site that is not the target site. Quantification can be performed by isolating a lesion and quantifying a number of immune cells, for example tumor infiltrating lymphocytes, comprising the enhancer receptor. Movement and/or trafficking of an immune cell comprising a switch molecule can be greater than that of a comparable immune cell lacking the enhancer receptor. In some embodiments, the number of immune cells comprising the enhancer receptor at a target site, for example a tumor lesion, can be about 5X, 10X, 15X, 20X, 25X, 30X, 35X, or 40X that of the number of comparable immune cells lacking the enhancer receptor. Trafficking can also be determined in vitro utilizing a transwell migration assay. In some embodiments, the number of immune cells comprising the enhancer receptor at a target site, for example in a transwell migration assay, can be about 5X, 10X, 15X, 20X, 25X, 30X, 35X, or 40X that of the number of comparable immune cells lacking the enhancer receptor.

In some embodiments, immune cell activation signal can comprise or result in exhaustion and/or activation of the immune cell. Exhaustion and/or activation of an immune cell can be determined by phenotypic analysis by flow cytometry or microscopic analysis. For example, expression levels of markers of exhaustion, for instance programmed cell death protein 1 (PD1), lymphocyte activation gene 3 protein (LAG3), 2B4, CD160, Tim3, and T cell immunoreceptor with immunoglobulin and ITIM domains (TIGIT), can be determined quantitatively and/or qualitatively. In some cases, immune cells, such as T cells, can lose effector functions in a hierarchical manner and become exhausted. As a result of exhaustion, functions such as IL-2 production and cytokine expression, as well as high proliferative capacity, can be lost. Exhaustion can also be followed by defects in the production of IFNγ, TNF and chemokines, as well as in degranulation. Exhaustion or activation of a modified immune cell provided herein can be greater than that of a comparable immune cell lacking the enhancer receptor. In some embodiments, the immune cell provided herein can undergo at least about a 1 fold, 2 fold, 3 fold, 4 fold, 5 fold, 6 fold, 7 fold, 8 fold, 9 fold, 10 fold, 11 fold, 12 fold, 13 fold, 14 fold, 15 fold, 20 fold, 30 fold, 40 fold, 50 fold, 60 fold, 70 fold, 80 fold, 90 fold, 100 fold, 150 fold, 200 fold, 250 fold, or over 300 increase in exhaustion or activation as compared to a comparable immune cell lacking the enhancer receptor. In some embodiments, the immune cell comprising provided herein can undergo at least about a 1 fold, 2 fold, 3 fold, 4 fold, 5 fold, 6 fold, 7 fold, 8 fold, 9 fold, 10 fold, 11 fold, 12 fold, 13 fold, 14 fold, 15 fold, 20 fold, 30 fold, 40 fold, 50 fold, 60 fold, 70 fold, 80 fold, 90 fold, 100 fold, 150 fold, 200 fold, 250 fold, or over 300 decrease in exhaustion or activation as compared to a comparable immune cell lacking the enhancer receptor.

In some embodiments, the binding of the target cells to enhanced receptors can generate immune cell activation signals in the immune cells modified with the enhanced receptors.

In some embodiments, upon binding of the enhancer receptor to the ligand, the enhancer receptor modified immune cell (e.g., modified TIL or modified T cell) exhibits enhanced neoantigen binding as compared to a comparable immune cell lacking the enhancer receptor.

In some embodiments, the immune cells (such as TIL, neoantigen-reactive T cells, CAR-T, TCR-T, NK, etc.) modified with the enhanced receptors can better overcome the inhibitory signal from tumor microenvironment to the immune cells to a certain extent as compared to immune cells without enhanced receptor modification (corresponding to the aforementioned TIL, neoantigen-reactive T cells, CAR-T, TCR-T, NK, etc.), wherein the inhibitory signal can be derived from ligand or antigen of the extracellular domain of the enhanced receptor, or can also be derived from inhibitory signal from the tumor microenvironments to the immune cells other than the ligand or antigen.

In some embodiments, the ECD of the enhanced receptor is PD1 or PDL1 monoclonal antibody, and the ICD is any kind of costimulatory molecule, wherein the tumor inhibitory signals that can be effectively overcome to a certain extent is not limited to immune cell inhibitory signals from PDL1, but can also include from CD47, TIM-3 ligands such as Galectin-9, TIGIT ligands such as CD155 and CD122 or PVR, CTLA-4 ligands such as B7.

In some embodiments, if the ECD of the enhanced receptor is SIRPα or CD47 monoclonal antibody, and the ICD is any kind of costimulatory molecule, wherein the tumor inhibitory signals that can be effectively overcome to a certain extent is not only immune cell inhibitory signals from CD47, but also from PDL1, TIM-3 ligands Such as Galectin-9, TIGIT ligands such as CD155 and CD122 or PVR, CTLA-4 ligands such as B77.

In some embodiments, if the ECD of the enhanced receptor is a monoclonal antibody of TIM-3 ligand such as a monoclonal antibody of Galectin-9 or a monoclonal antibody of TIM-3, and the ICD is any kind of costimulatory molecule, wherein the tumor inhibitory signals that can be effectively overcome to a certain extent is not only immune cell inhibitory signals from TIM-3 ligand such as Galectin-9, but also from PDL1, TIGIT ligands such as CD155 and CD122 or PVR, CTLA-4 ligands such as B77.

In some embodiments, if the ECD of the enhanced receptor is a monoclonal antibody of TIGIT or its ligand such as CD155 monoclonal antibody or CD122 monoclonal antibody or PVR monoclonal antibody, and the ICD is any kind of costimulatory molecule, wherein the tumor inhibitory signals that can be effectively overcome to a certain extent is not only immune cell inhibitory signals from TIGIT ligands such as CD155 and CD122 or PVR, but also from PDL1, TIM-3 ligands such as Galectin-9, CTLA-4 ligands such as B77.

In some embodiments, if the ECD of the enhanced receptor is VISTA, and the ICD is any kind of costimulatory molecule, wherein the tumor inhibitory signals that can be effectively overcome to a certain extent is not only immune cell inhibitory signals from VISTA, but also from PDL1, CD47, TIM-3 ligands such as Galectin-9, TIM-3 ligands such as Galectin-9, , TIGIT ligands such as CD155 and CD122 or PVR, CTLA-4 ligands such as B77.

In some embodiment, T cells modified by aBA-CAR and receptor enhancer further carry artificial (or exogenous) T cell receptor (TCR) or a different chimeric antigen receptor CAR, when TCR or CAR can recognize target cells, T cells modified with enhanced receptors have greater activation function of immune cells as compared to T cells without modification with the enhanced receptors; when neither artificial TCR nor the different CAR can recognize target cells, T cells modified with enhanced receptors do not have greater activation function of immune cells as compared to T cells that have not been modified with enhanced receptors.

The T cell receptor (TCR) complex that exhibits specific binding to the neoantigen may be an endogenous TCR complex or an exogenous TCR complex. The TCR complex (e.g., endogenous or exogenous) of the modified immune cell can confer antigen binding specificity (e.g., neoantigen binding) to the immune cell.

In one aspect, the present disclosure provides modified immune cells specifically binding to a neoantigen, wherein the modified immune cells comprise: (1) a chimeric stimulating molecule, comprising a polypeptide extracellular domain (PED) that binds to the neoantigen, wherein the PED is fused to an intracellular domain (ICD) of a co-stimulatory molecule that mediates an immune cell activation signal, and binding of the chimeric stimulating molecule to the neoantigen yields the immune cell activation signal in the modified immune cells; and (b) aBA-CAR, comprising (i) an interacting domain capable of binding the booster antigen; (ii) a transmembrane domain; (iii) an intracellular signaling domain. In some embodiments, PED can be the extracellular domain of surface protein of unmodified TIL. In some embodiments, examples of PED include antibody, derivatives, variants and fragments thereof.

In one embodiments, the present disclosure provides modified immune cells that specifically bind to neoantigens or tumor-associated antigens (TAA) or cancer testis antigens, or antigens derived from viruses (such as HPV and EBV), wherein the modified immune cells comprise: (a) natural TCR that can specifically bind to neoantigens or genetically engineered exogenous TCR; (b) an enhanced receptor comprising the extracellular domain (ECD) of a protein, wherein the ECD is fused to an intracellular domain (ICD) of a co-stimulatory molecule that mediates an immune cell activation signal, and binding of the enhanced receptor to the ligand yields the immune cell activation signal in the modified immune cells instead of the immune cell inactivation signal, and (c) aBA-CAR, comprising (i) an interacting domain capable of binding the booster antigen; (ii) a transmembrane domain; (iii) an intracellular signaling domain; in the above three of a, b, c, c is indispensable, in some embodiments, only one of b and a may exist; in some embodiments, all three of a, b, and c coexist.

In an embodiment, the present disclosure provides a modified tumor infiltrating lymphocyte (TIL) that specifically binds to a neoantigen, wherein the modified immune cell comprises: (a) a natural TCR capable of binding the neoantigen; (b) an enhanced receptor comprising an extracellular domain (ECD) of a protein, wherein the ECD is fused to an intracellular domain (ICD) of a co-stimulatory molecule that mediates an immune cell activation signal, and wherein the molecule is converted to ligand, yielding immune cell activation signal in the modified TIL instead of the immune cell inactivation signal, and (c) a aBA-CAR comprising (i) an interacting domain capable of binding a booster antigen; (ii) a transmembrane domain; and (iii) an intracellular signaling domain. Among the above three of a, b, and c, c is indispensable; in some embodiments, only one of b or a may exist; in some embodiments, a, b, and c coexist, and the cells in which the three coexist are called Super TIL (STIL).

In an aspect, the present disclosure provides a modified immune cell overexpressing a cytokine, for example a chemokine, wherein the immune cell is (i) a tumor infiltrating lymphocyte (TIL); (ii) a stromal tumor infiltrating lymphocyte (sTIL); or (iii) a T cell exhibiting specific binding to an antigen. The modified immune cell overexpressing the chemokine can be any modified immune cell provided herein.

Cytokines refer to proteins (e.g., chemokines, interferons, lymphokines, interleukins, and tumor necrosis factors) released by cells which can affect cell behavior. Cytokines are produced by a broad range of cells, including immune cells such as macrophages, B lymphocytes, T lymphocytes and mast cells, as well as endothelial cells, fibroblasts, and various stromal cells. A given cytokine can be produced by more than one type of cell. Cytokines can be involved in producing systemic or local immunomodulatory effects.

Certain cytokines can function as pro-inflammatory cytokines. Pro-inflammatory cytokines refer to cytokines involved in inducing or amplifying an inflammatory reaction. Pro-inflammatory cytokines can work with various cells of the immune system, such as neutrophils and leukocytes, to generate an immune response. Certain cytokines can function as anti-inflammatory cytokines. Anti-inflammatory cytokines refer to cytokines involved in the reduction of an inflammatory reaction. Anti-inflammatory cytokines, in some cases, can regulate a pro-inflammatory cytokine response. Some cytokines can function as both pro- and anti-inflammatory cytokines. Certain cytokines, e.g., chemokines, can function in chemotaxis. Chemokines can induce directed chemotaxis in nearby responsive cells.

In some embodiments, the expression of a cytokine having pro-inflammatory and/or chemotactic functions can be up-regulated in an immune cell. Up-regulating the expression of a cytokine having pro-inflammatory and/or chemotactic functions can be useful, for example, to stimulate an immune response against a target cell in immunotherapy.

Examples of cytokines that can be overexpressed by immune cells provided herein include, but are not limited to lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha; mullerian-inhibiting substance; mouse gonadotropin-associated peptide; inhibin; activin; vascular endothelial growth factor; integrin; thrombopoietin (TPO); nerve growth factors such as NGF-alpha; platelet-growth factor; transforming growth factors (TGFs) such as TGF-alpha, TGF-beta, TGF-beta1, TGF-beta2, and TGF-beta3; insulin-like growth factor-I and -II; erythropoietin (EPO); FLT-3L; stem cell factor (SCF); osteoinductive factors; interferons (IFNs) such as IFN-α, IFN-β, IFN-γ; colony stimulating factors (CSFs) such as macrophage-CSF (M-CSF); granulocyte-macrophage-CSF (GM-CSF); granulocyte-CSF (G-CSF); macrophage stimulating factor (MSP); interleukins (ILs) such as IL-1, IL-1a, IL-1b, IL-1RA, IL-18, IL-2, IL-3, IL-4, IL-5, IL-6, IL-7, IL-8, IL-9, IL-10, IL-11, IL-12, IL-13, IL-14, IL-15, IL-16, IL-17, IL-20; a tumor necrosis factor such as CD154, LT-beta, TNF-alpha, TNF-beta, 4-1BBL, APRIL, CD70, CD153, CD178, GITRL, LIGHT, OX40L, TALL-1, TRAIL, TWEAK, TRANCE; and other polypeptide factors including LIF, oncostatin M (OSM) and kit ligand (KL). Cytokine receptors refer to the receptor proteins which bind cytokines. Cytokine receptors may be both membrane-bound and soluble.

In some embodiments, the overexpressed cytokine is an interleukin (IL-1) family member (e.g., ligand), an IL-1 receptor family member, an interleukin-6 (IL-6) family member (e.g., ligand), an IL-6 receptor, an interleukin-10 (IL-10) family member (e.g., ligand), an IL-10 receptor, an interleukin-12 (IL-12) family member (e.g., ligand), an IL-12 receptor, an interleukin-17 (IL-17) family member (e.g., ligand), or an IL-17 receptor.

In some embodiments, the overexpressed cytokine is an interleukin-1 (IL-1) family member or related protein; a tumor necrosis factor (TNF) family member or related protein; an interferon (IFN) family member or related protein; an interleukin-6 (IL-6) family member or related protein; or a chemokine or related protein. In some embodiments, the cytokine is selected from IL18, IL18BP, ILIA, IL1B, IL1F10, IL1F3/IL1RA, IL1F5, IL1F6, IL1F7, IL1F8, IL1RL2, IL1F9, IL33, BAFF/BLyS/TNFSF138, 4-1BBL, CD153/CD30L/TNFSF8, CD40LG, CD70, Fas Ligand/FASLG/CD95L/CD178, EDA-A1, TNFSF14/LIGHT/CD258, TNFA, LTA/TNFB/TNFSF1, LTB/TNFC, CD70/CD27L/TNFSF7, TNFSF10/TRAIL/APO-2L(CD253), RANKL/OPGL/TNFSF11(CD254), TNFSF12, TNF-alpha/TNFA,TNFSF13, TL1A/TNFSF15, OX-40L/TNFSF4/CD252, CD40L/CD154/TNFSF5, IFNA1, IFNA10, IFNA13, IFNA14, IFNA2, IFNA4, IFNA7, IFNB1, IFNE, IFNG, IFNZ, IFNA8, IFNA5/IFNaG, IPNω/IFNW1, CLCF1, CNTF, IL11, IL31, IL6, Leptin, LIF, OSM, CCL1/TCA3, CCL11, CCL12/MCP-5,CCL13/MCP-4, CCL14, CCL15, CCL16, CCL17/TARC, CCL18, CCL19, CCL2/MCP-1, CCL20, CCL21, CCL22/MDC, CCL23, CCL24, CCL25, CCL26, CCL27, CCL28, CCL3, CCL3L3, CCL4, CCL4L1/LAG-1, CCL5, CCL6, CCL7, CCL8, CCL9, CX3CL1, CXCL1, CXCL10, CXCL11, CXCL12, CXCL13, CXCL14, CXCL15, CXCL16, CXCL17, CXCL2/MIP-2, CXCL3, CXCL4, CXCL5, CXCL6, CXCL7/Ppbp, CXCL9, IL8/CXCL8, XCL1, XCL2, FAM19A1, FAM19A2, FAM19A3, FAM19A4, and FAM19A5.

Cytokine expression can be evaluated using a variety of methods. Cytokine expression can be evaluated by assaying cell culture media (e.g., *in vitro* production) in which the modified immune cells are grown or sera (e.g., *in vivo* production) obtained from a subject having the modified immune cells for the presence of one or more cytokines. Cytokine levels can be quantified in various suitable units, including concentration, using any suitable assay. In some embodiments, cytokine protein is detected. In some embodiments, mRNA transcripts of cytokines are detected. Examples of cytokine assays include enzyme-linked immunosorbent assays (ELISA), immunoblot, immunofluorescence assays, radioimmunoassays, antibody arrays which allow various cytokines in a sample to be detected in parallel, bead-based arrays, quantitative PCR, microarray, etc. Other suitable methods may include proteomics approaches (2-D gels, MS analysis etc).

In some embodiments, the cytokine overexpressed by a modified immune cell provided herein is a chemokine. The chemokine can be, for example, a CC chemokine, a CXC chemokine, a C chemokine, and a CX3C chemokine. In some embodiments, the chemokine overexpressed by a modified immune cell is a CC chemokine selected from CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, and CCL28. said chemokine is a CXC chemokine selected from CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, and CXCL17. In some embodiments, the chemokine overexpressed by a modified immune cell is a C chemokine selected from XCL1 and XCL2. In some embodiments, the chemokine overexpressed by an immune cell is a CX3C chemokine, and the CX3C chemokine is CX3CL1.

In some cases, herein provided is an immune cell, which is a NK cell with gene modification other than aBA-CAR modification.

In some cases, herein provided is NK cells, which is a CAR-NK cell with a CAR gene modification other than aBA-CAR modification, and the CAR gene modification other than aBA-CAR modification confers to the NK cells with a property of specifically recognizing and killing target cells; said CAR gene modification other than aBA-CAR modification can include a part of the NKG2D structure.

In some cases, booster adjuvant is a cascade booster system composed of n kinds of booster cells of the present invention, wherein n is a positive integer, and the first-grade booster cells express the first booster antigen (BA1), the second-grade booster cells express the second booster antigen (BA2) and aBA1-CAR specifically targeting the first booster antigen, and the third-grade booster cells express the third booster antigen (BA3) and aBA2-CAR specifically targeting the second booster antigen, ... the n^{th} booster cells express the n^{th} booster antigen (BAn) and aBAₙ₋₁-CAR targeting the n-1 booster antigen, said n^{th} booster antigen BAn can be recognized by the therapeutic immune cells, and booster antigens of different grades are different from each other.

In some cases, the number of stages, n, of the cascaded multi-stage boost system can be equal to any positive integer in the range of 1-10000000.

In some cases, in the multi-stage booster system of cascade amplification, starting from the second stage, the booster cells can be activated and expanded by the booster antigen from the previous stage through BA-CAR *in vivo.* The booster cells of different stages can be the cells of the same type (such as all T cells, or NK cells), or cells of different types, or a mixture of multiple types of cells.

The present disclosure provides an adjuvant pharmaceutical composition for immune cell therapy, characterized in that the pharmaceutical composition comprises the booster adjuvant described herein.

The present disclosure provides a pharmaceutical combination for immune cell therapy, characterized in that the pharmaceutical combination comprises the booster adjuvant herein, and the therapeutic immune cells of the present invention, wherein the therapeutic immune cells and the pharmaceutical composition are infused back in a single or consecutive multiple times at appropriate time points, so that the therapeutic immune cells can be activated by the adjuvant in the pharmaceutical composition and are quantitatively expanded, thus identifying and killing the target cells.

The present invention provides a method for treating cancer, characterized in using the adjuvant pharmaceutical composition for immune cell therapy or the pharmaceutical combination for immune cell therapy described herein, specifically, the therapeutic immune cell and the adjuvant drug for immune cell therapy being infused back in a single or consecutive multiple times at an appropriate time points.

In an aspect, the present disclosure provides a method of treating a cancer of a subject, comprising: (a) administering to a subject a modified TIL, a modified T cell obtained from positive screening of peripheral blood PBMC by one or more of PD1, TIM3, CD137, CD39, a modified T cell or a modified immune cell of any one of the various embodiments of the aspects herein; and (b) contacting a target cell of the cancer expressing a neoantigen with the modified TIL, modified T cell obtained by screening, modified T cell or modified immune cell under conditions that induces cytotoxicity of the modified TIL, modified T cell obtained by screening, modified T cell, or modified immune cell against the target cell of the cancer, thereby inducing death of the target cell of the cancer.

In an aspect, the present disclosure provides a method of expanding a T cell population, the method comprising: (a) providing a population of T cells comprising at least a modified immune cell of any one of the various embodiments of the aspects herein; (b) exposing the population of T cells to a booster antigen to effect expansion of the population of T cells.

In an aspect, the present disclosure provides a method of expanding a T cell population, comprising: (a) introducing a nucleic acid encoding aBA-CAR into the T cell population, thereby producing aBA-CAR-expressing cell or cell population, wherein the aBA-CAR comprises (i) an antigen interacting domain capable of binding CAR; (ii) a transmembrane domain; (iii) an intracellular signaling domain; and (b) contacting the aBA-CAR-expressing cell population with booster antigen, thereby producing an expanded and/or activated immune cell population. Wherein, the antigen is not specifically expressed by tumor target cells.

In an aspect, the present disclosure provides a composition comprising one or more polynucleotides that encodes one or more of: (a) an enhanced receptor, wherein the enhanced receptor comprises an extracellular domain (ECD) of a protein, wherein the ECD is fused to an intracellular domain (ICD) of co-stimulatory protein that mediates an immune cell activation signal; and (b) an antigen specific T cell receptor complex, or one or more components thereof.

In an aspect, the present disclosure provides a composition comprising one or more polynucleotides that encodes one or more of: (a) an antigen specific T cell receptor complex, or one or more components thereof; (b) aBA-CAR, comprising (i) an interacting domain capable of binding booster antigen; (ii) transmembrane domain; (iii) an intracellular signaling domain.

In an aspect, the present disclosure provides a composition comprising one or more polynucleotides that encodes one or more of: (a) an enhanced receptor, wherein the enhanced receptor comprises an extracellular domain (ECD) of a protein, wherein the ECD is fused to an intracellular domain (ICD) of co-stimulatory protein that mediates an immune cell activation signal; (b) aBA-CAR, comprising (i) an antigen interacting domain capable of binding a B cell surface protein; (ii) a transmembrane domain; and (iii) an intracellular signaling domain.

In an aspect, the present disclosure provides a composition comprising one or more polynucleotides that encodes one or more of: (a) an enhanced receptor, wherein the enhanced receptor comprises an extracellular domain (ECD) of a protein, wherein the ECD is fused to an intracellular domain (ICD) of co-stimulatory protein that mediates an immune cell activation signal; (b) an antigen specific T cell receptor complex, or one or more components thereof; and (c) aBA-CAR, comprising (i) an antigen interacting domain capable of binding a B cell surface protein; (ii) a transmembrane domain; and (iii) an intracellular signaling domain.

In various embodiments of the aspects herein, promoters that can be used with the compositions of the present disclosure include those active in a eukaryotic, mammalian, non-human mammalian or human cell. The promoter can be an inducible or constitutively active promoter. Alternatively or additionally, the promoter can be tissue or cell specific.

Non-limiting examples of suitable eukaryotic promoters (i.e. promoters functional in a eukaryotic cell) can include those from cytomegalovirus (CMV) immediate early, herpes simplex virus (HSV) thymidine kinase, early and late SV40, long terminal repeats (LTRs) from retrovirus, human elongation factor-1 promoter (EF1), a hybrid construct comprising the cytomegalovirus (CMV) enhancer fused to the chicken beta-active promoter (CAG), murine stem cell virus promoter (MSCV), phosphoglycerate kinase-1 locus promoter (PGK) and mouse metallothionein-I. The promoter can be a fungi promoter. The promoter can be a plant promoter. A database of plant promoters can be found (e.g., PlantProm). The expression vector may also contain a ribosome binding site for translation initiation and a transcription terminator. The expression vector may also include appropriate sequences for amplifying expression.

In various embodiments of the aspects herein, modified immune cells can specifically bind a neoantigen and/or a neoepitope. Neoantigens and neoepitopes generally refer to tumor-specific mutations that in some cases trigger an antitumor T cell response. For example, these endogenous mutations can be identified using a whole-exomic-sequencing approach (Tran E, et al., "Cancer immunotherapy based on mutation-specific CD4+ T cells in a patient with epithelial cancer," Science 344: 641-644 (2014)). An modified immune cell (e.g., modified TIL or modified T cell) comprising an enhanced receptor can exhibit specific binding to a tumor-specific neo-antigen. Neoantigens bound by the immune cell can be expressed on a target cell, and for example, are encoded by mutations in an endogenous gene. In some cases, a neoantigen or neoepitope specifically bound by an immune cell can be encoded by a mutated gene. The gene can be selected from the group consisting of: ABL1, ACOl 1997, ACVR2A, AFP, AKT1, ALK, ALPPL2, ANAPC1, APC, ARID1A, AR, AR-v7, ASCL2, β2M, BRAF, BTK, C15ORF40, CDH1, CLDN6, CNOT1, CT45A5, CTAG1B (encodes NY-ESO-1), DCT, DKK4,EEF1B2, EEF1DP3, EGFR, EIF2B3, env, EPHB2, ERBB3, ESR1, ESRP1, FAM11 IB, FGFR3, FRG1B,GAGE1, GAGE 10, GATA3, GBP3, HER2, IDH1, JAK1, KIT, KRAS, LMAN1, MABEB 16, MAGEA1,MAGEA10, MAGEA4, MAGEA8, MAGEB 17, MAGEB4, MAGEC1, MEK, MLANA, MLL2, MMP13,MSH3, MSH6, MYC, NDUFC2, NRAS, NY-ESO, PAGE2, PAGE5, PDGFRa, PIK3CA, PMEL, pol protein, POLE,PTEN, RAC1, RBM27, RNF43, RPL22, RUNX1, SEC31A, SEC63, SF3B 1, SLC35F5, SLC45A2, SMAP1, SMAP1, SPOP, TFAM, TGFBR2, THAP5, TP53, TTK, TYR, UBR5, VHL, and XPOT. In some embodiments, the neoantigen is selected based on a genetic profile of a tumor sample from an individual. In some embodiments, the neoantigen may be selected based on a somatic mutation profile of a tumor sample from an individual.

In various embodiments of the aspects herein, a modified immune cell further comprises a kill switch (or suicide switch). A kill switch can be activated to eliminate the immune cell in cases of severe toxicity, such as hypercytokinemia. This can happen when the immune system has such a strong response that many inflammatory cytokines are released, triggering mild to severe symptoms including fever, headache, rash, rapid heartbeat, low blood pressure, and trouble breathing. A kill switch can be a drug-inducible kill-switch. The kill switch can comprise an inducible caspase 9.

Various embodiments of the aspects herein comprise a cell, for example a modified immune cell. Cells, for example immune cells (e.g., lymphocytes including T cells and NK cells), can be obtained from a subject. Non-limiting examples of subjects include humans, dogs, cats, mice, rats, and transgenic species thereof. Examples of samples from a subject from which cells can be derived include, without limitation, skin, heart, lung, kidney, bone marrow, breast, pancreas, liver, muscle, smooth muscle, bladder, gall bladder, colon, intestine, brain, prostate, esophagus, thyroid, serum, saliva, urine, gastric and digestive fluid, tears, stool, semen, vaginal fluid, interstitial fluids derived from tumorous tissue, ocular fluids, sweat, mucus, earwax, oil, glandular secretions, spinal fluid, hair, fingernails, plasma, nasal swab or nasopharyngeal wash, spinal fluid, cerebral spinal fluid, tissue, throat swab, biopsy, placental fluid, amniotic fluid, cord blood, emphatic fluids, cavity fluids, sputum, pus, microbiota, meconium, breast milk, and/or other excretions or body tissues.

In some cases, a cell can be a population of T cells, NK cell, B cells, and the like obtained from a subject. T cells can be obtained from a number of sources, including PBMCs, bone marrow, lymph node tissue, cord blood, thymus tissue, and tissue from a site of infection, ascites, pleural effusion, spleen tissue, and tumors. In some embodiments, T cells can be obtained from a unit of blood collected from a subject using any number of techniques, such as FicollTM separation. In one embodiment, cells from the circulating blood of an individual are obtained by apheresis. The apheresis product typically contains lymphocytes, including T cells, monocytes, granulocytes, B cells, other nucleated white blood cells, red blood cells, and platelets. The cells collected by apheresis may be washed to remove the plasma fraction and to place the cells in an appropriate buffer or media for subsequent processing steps.

Any of a variety of immune cells can be utilized in the aspects herein. In some embodiments, immune cells comprise granulocytes such as asophils, eosinophils, and neutrophils; mast cells; monocytes which can develop into macrophages; antigen-presenting cells such as dendritic cells; and lymphocytes such as natural killer cells (NK cells), B cells, and T cells. In some embodiments, an immune cell is an immune effector cell. An immune effector cell refers to an immune cell that can perform a specific function in response to a stimulus. In some embodiments, an immune cell is an immune effector cell which can induce cell death. In some embodiments, the immune cell is a lymphocyte. In some embodiments, the lymphocyte is a NK cell. In some embodiments the lymphocyte is a T cell. In some embodiments, the T cell is an activated T cell. T cells include both naive and memory cells (e.g. central memory or TCM, effector memory or TEM and effector memory RA or TEMRA), effector cells (e.g. cytotoxic T cells or CTLs or Tc cells), helper cells (e.g. Thl, Th2, Th3, Th9, Th7, TFH), regulatory cells (e.g. Treg, and Trl cells), natural killer T cells (NKT cells), tumor infiltrating lymphocytes (TILs), lymphocyte-activated killer cells (LAKs), αβ T cells, γδ T cells, and similar unique classes of the T cell lineage. T cells can be divided into two broad categories: CD8+ T cells and CD4+ T cells, based on which protein is present on the cell's surface. T cells expressing a subject system can carry out multiple functions, including killing infected cells and activating or recruiting other immune cells. CD8+ T cells are referred to as cytotoxic T cells or cytotoxic T lymphocytes (CTLs). CTLs expressing a subject system can be involved in recognizing and removing virus-infected cells and cancer cells. CTLs have specialized compartments, or granules, containing cytotoxins that cause apoptosis, e.g., programmed cell death. CD4+ T cells can be subdivided into four sub-sets - Th1, Th2, Thl7, and Treg, with "Th" referring to "T helper cell," although additional sub-sets may exist. Th1 cells can coordinate immune responses against intracellular microbes, especially bacteria. They can produce and secrete molecules that alert and activate other immune cells, like bacteria-ingesting macrophages. Th2 cells are involved in coordinating immune responses against extracellular pathogens, like helminths (parasitic worms), by alerting B cells, granulocytes, and mast cells. Th17 cells can produce interleukin 17 (IL-17), a signaling molecule that activates immune and non-immune cells. Th17 cells are important for recruiting neutrophils.

In some embodiments, a population of immune cells provided herein can be heterogeneous. In some embodiments, cells used can be composed of a heterogeneous mixture of CD4 and CD8 T cells. The CD4 and CD8 cells can have phenotypic characteristics of circulating effector T cells. Said CD4 and CD8 cells can also have a phenotypic characteristic of effector-memory cells. In some embodiment, cells can be central-memory cells.

In some embodiments, cells include peripheral blood mononuclear cells (PBMC), peripheral blood lymphocytes (PBLs), and other blood cell subsets such as, but not limited to, T cell, a natural killer cell, a monocyte, a natural killer T cell, a monocyte-precursor cell, a hematopoietic stem cell or a non-pluripotent stem cell. In some cases, the cell can be any immune cell, including any T-cell such as tumor infiltrating cells (TILs), such as CD3+ T-cells, CD4+ T-cells, CD8+ T-cells, or any other type of T-cell. The T cell can also include memory T cells, memory stem T cells, or effector T cells. The T cells can also be selected from a bulk population, for example, selecting T cells from whole blood. The T cells can also be expanded from a bulk population. The T cells can also be skewed towards particular populations and phenotypes. For example, the T cells can be skewed to phenotypically comprise, CD45RO (-), CCR7 (+), CD45RA (+), CD62L (+), CD27 (+), CD28 (+) and/or IL-7Rα (+). Suitable cells can be selected that comprise one of more markers selected from a list comprising: CD45RO (-), CCR7 (+), CD45RA (+), CD62L (+), CD27 (+), CD28 (+) and/or IL-7Rα (+). Cells also include stem cells such as, by way of example, embryonic stem cells, induced pluripotent stem cells, hematopoietic stem cells, neuronal stem cells and mesenchymal stem cells. Cells can comprise any number of primary cells, such as human cells, non-human cells, and/or mouse cells. Cells can be progenitor cells. Cells can be derived from the subject to be treated (e.g., patient). Cells can be derived from a human donor. Host cells can be stem memory TSCM cells comprised of CD45RO (-), CCR7(+), CD45RA (+), CD62L+ (L-selectin), CD27+, CD28+ and IL-7Rα+, said stem memory cells can also express CD95, IL-2Rβ, CXCR3, and LFA-1, and show numerous functional attributes distinctive of said stem memory cells. Host cells can be central memory TCM cells comprising L-selectin and CCR7, said central memory cells can secrete, for example, IL-2, but not IPNγ or IL-4. Cells can also be effector memory TEM cells comprising L-selectin or CCR7 and produce, for example, effector cytokines such as IPNγ and IL-4.

In various embodiments of the aspects herein, an immune cell comprises a lymphocyte. In some embodiments, the lymphocyte is a natural killer cell (NK cell). In some embodiments, the lymphocyte is a T cell. T cells can be obtained from a number of sources, including peripheral blood mononuclear cells, bone marrow, lymph node tissue, spleen tissue, umbilical cord, and tumors. In some embodiments, any number of T cell lines available can be used. Immune cells such as lymphocytes (e.g., cytotoxic lymphocytes) can preferably be autologous cells, although heterologous cells can also be used. T cells can be obtained from a unit of blood collected from a subject using any number of techniques, such as Ficoll separation. Cells from the circulating blood of an individual can be obtained by apheresis or leukapheresis. The apheresis product typically contains lymphocytes, including T cells, monocytes, granulocytes, B cells, other nucleated white blood cells, red blood cells, and platelets. The cells collected by apheresis can be washed to remove the plasma fraction and to place the cells in an appropriate buffer or media, such as phosphate buffered saline (PBS), for subsequent processing steps. After washing, the cells can be resuspended in a variety of biocompatible buffers, such as Ca-free, Mg-free PBS. Alternatively, the undesirable components of the apheresis sample can be removed and the cells directly resuspended in culture media. Samples can be provided directly by the subject, or indirectly through one or more intermediaries, such as a sample collection service provider or a medical provider (e.g. a physician or nurse). In some embodiments, isolating T cells from peripheral blood leukocytes can include lysing the red blood cells and separating peripheral blood leukocytes from monocytes by, for example, centrifugation through, e.g., a PERCOL^{™} gradient.

A specific subpopulation of T cells, such as CD4+ or CD8+ T cells, can be further isolated by positive or negative selection techniques. Negative selection of a T cell population can be accomplished, for example, with a combination of antibodies directed to surface markers unique to the cells negatively selected. One suitable technique includes cell sorting via negative magnetic immunoadherence, which utilizes a cocktail of monoclonal antibodies directed to cell surface markers present on the cells negatively selected. For example, to isolate CD4+ cells, a monoclonal antibody cocktail can include antibodies to CD14, CD20, CD1 lb, CD16, HLA-DR, and CD8. The process of negative selection can be used to produce a desired T cell population that is primarily homogeneous. In some embodiments, a composition comprises a mixture of two or more (e.g. 2, 3, 4, 5, or more) different kind of T-cells.

In some embodiments, the immune cell is a member of an enriched population of cells. One or more desired cell types can be enriched by any suitable method, non-limiting examples of which include treating a population of cells to trigger expansion and/or differentiation to a desired cell type, treatment to stop the growth of undesired cell type(s), treatment to kill or lyse undesired cell type(s), purification of a desired cell type (e.g. purification on an affinity column to retain desired or undesired cell types on the basis of one or more cell surface markers). In some embodiments, the enriched population of cells is a population of cells enriched in cytotoxic lymphocytes selected from cytotoxic T cells (also variously known as cytotoxic T lymphocytes, CTLs, T killer cells, cytolytic T cells, CD8+ T cells, and killer T cells), natural killer (NK) cells, and lymphokine- activated killer (LAK) cells.

For isolation of a desired population of cells by positive or negative selection, the concentration of cells and surface (e.g., particles such as beads) can be varied. In certain embodiments, it can be desirable to significantly decrease the volume in which beads and cells are mixed together (i.e., increase the concentration of cells), to ensure maximum contact of cells and beads. For example, a concentration of 2 billion cells/mL can be used. In some embodiments, a concentration of 1 billion cells/mL is used. In some embodiments, greater than 100 million cells/mL are used. A concentration of cells of 10, 15, 20, 25, 30, 35, 40, 45, or 50 million cells/mL can be used. In yet another embodiment, a concentration of cells from 75, 80, 85, 90, 95, or 100 million cells/mL can be used. In further embodiments, concentrations of 125 or 150 million cells/mL can be used. Using high concentrations can result in increased cell yield, cell activation, and cell expansion.

A variety of target cells can be killed using the systems and methods of the subject disclosure. A target cell to which this method can be applied includes a wide variety of cell types. A target cell can be *in vitro.* A target cell can be *in vivo.* A target cell can be *ex vivo.* A target cell can be an isolated cell. A target cell can be a cell inside of an organism. A target cell can be an organism. A target cell can be a cell in a cell culture. A target cell can be one of a collection of cells. A target cell can be a mammalian cell or derived from a mammalian cell. A target cell can be a rodent cell or derived from a rodent cell. A target cell can be a human cell or derived from a human cell. A target cell can be a prokaryotic cell or derived from a prokaryotic cell. A target cell can be a bacterial cell or can be derived from a bacterial cell. A target cell can be an archaeal cell or derived from an archaeal cell. A target cell can be a eukaryotic cell or derived from a eukaryotic cell. A target cell can be a pluripotent stem cell. A target cell can be a plant cell or derived from a plant cell. A target cell can be an animal cell or derived from an animal cell. A target cell can be an invertebrate cell or derived from an invertebrate cell. A target cell can be a vertebrate cell or derived from a vertebrate cell. A target cell can be a microbe cell or derived from a microbe cell. A target cell can be a fungi cell or derived from a fungi cell. A target cell can be from a specific organ or tissue.

A target cell can be a stem cell or progenitor cell. Target cells can include stem cells (e.g., adult stem cells, embryonic stem cells, induced pluripotent stem (iPS) cells) and progenitor cells (e.g., cardiac progenitor cells, neural progenitor cells, etc.). Target cells can include mammalian stem cells and progenitor cells, including rodent stem cells, rodent progenitor cells, human stem cells, human progenitor cells, etc. Clonal cells can comprise the progeny of a cell. A target cell can comprise a target nucleic acid. A target cell can be in a living organism. A target cell can be a genetically modified cell. A target cell can be a host cell.

A target cell can be a primary cell. For example, cultures of primary cells can be passaged 0 times, 1 time, 2 times, 4 times, 5 times, 10 times, 15 times or more. Cells can be unicellular organisms. Cells can be grown in culture.

A target cell can be a diseased cell. A diseased cell can have altered metabolic, gene expression, and/or morphologic features. A diseased cell can be a cancer cell, a diabetic cell, and a apoptotic cell. A diseased cell can be a cell from a diseased subject. Exemplary diseases can include blood disorders, cancers, metabolic disorders, eye disorders, organ disorders, musculoskeletal disorders, cardiac disease, and the like.

If the target cells are primary cells, they may be harvested from an individual by any method. For example, leukocytes may be harvested by apheresis, leukocytapheresis, density gradient separation, etc. Cells from tissues such as skin, muscle, bone marrow, spleen, liver, pancreas, lung, intestine, stomach, etc. can be harvested by biopsy. An appropriate solution may be used for dispersion or suspension of the harvested cells. Such solution can generally be a balanced salt solution, (e.g. normal saline, phosphate-buffered saline (PBS), Hank's balanced salt solution, etc.), conveniently supplemented with fetal calf serum or other naturally occurring factors, in conjunction with an acceptable buffer at low concentration. Buffers can include HEPES, phosphate buffers, lactate buffers, etc. Cells may be used immediately, or they may be stored (e.g., by freezing). Frozen cells can be thawed and can be capable of being reused. Cells can be frozen in a DMSO, serum, medium buffer (e.g., 10% DMSO, 50% serum, 40% buffered medium), and/or some other such common solution used to preserve cells at freezing temperatures.

Non-limiting examples of cells which can be target cells include, but are not limited to, lymphoid cells, such as B cell, T cell (Cytotoxic T cell, Natural Killer T cell, Regulatory T cell, T helper cell), Natural killer cell, cytokine induced killer (CIK) cells (see e.g. US20080241194); myeloid cells, such as granulocytes (Basophil granulocyte, Eosinophil granulocyte, Neutrophil granulocyte/Hypersegmented neutrophil), Monocyte/Macrophage, Red blood cell (Reticulocyte), Mast cell, Thrombocyte/Megakaryocyte, Dendritic cell; cells from the endocrine system, including thyroid (Thyroid epithelial cell, Parafollicular cell), parathyroid (Parathyroid chief cell, Oxyphil cell), adrenal (Chromaffin cell), pineal (Pinealocyte) cells; cells of the nervous system, including glial cells (Astrocyte, Microglia), Magnocellular neurosecretory cell, Stellate cell, Boettcher cell, and pituitary (Gonadotrope, Corticotrope, Thyrotrope, Somatotrope, Lactotroph ); cells of the Respiratory system, including Pneumocyte (Type I pneumocyte, Type II pneumocyte), Clara cell, Goblet cell, Dust cell; cells of the circulatory system, including Myocardiocyte, Pericyte; cells of the digestive system, including stomach (Gastric chief cell, Parietal cell), Goblet cell, Paneth cell, G cells, D cells, ECL cells, I cells, K cells, S cells; enteroendocrine cells, including enterochromaffm cell, APUD cell, liver (Hepatocyte, Kupffer cell), Cartilage/bone/muscle; bone cells, including Osteoblast, Osteocyte, Osteoclast, teeth (Cementoblast, Ameloblast); cartilage cells, including Chondroblast, Chondrocyte; skin cells, including Trichocyte, Keratinocyte, Melanocyte (Nevus cell); muscle cells, including Myocyte; urinary system cells, including Podocyte, Juxtaglomerular cell, Intraglomerular mesangial cell/Extraglomerular mesangial cell, Kidney proximal tubule brush border cell, Macula densa cell; reproductive system cells, including Spermatozoon, Sertoli cell, Leydig cell, Ovum; and other cells, including Adipocyte, Fibroblast, Tendon cell, Epidermal keratinocyte (differentiating epidermal cell), Epidermal basal cell (stem cell), Keratinocyte of fingernails and toenails, Nail bed basal cell (stem cell), Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, Hair matrix cell (stem cell), Wet stratified barrier epithelial cells, Surface epithelial cell of stratified squamous epithelium of cornea, tongue, oral cavity, esophagus, anal canal, distal urethra and vagina, basal cell (stem cell) of epithelia of cornea, tongue, oral cavity, esophagus, anal canal, distal urethra and vagina, Urinary epithelium cell (lining urinary bladder and urinary ducts), Exocrine secretory epithelial cells, Salivary gland mucous cell (polysaccharide-rich secretion), Salivary gland serous cell (glycoprotein enzyme -rich secretion), Von Ebner's gland cell in tongue (washes taste buds), Mammary gland cell (milk secretion), Lacrimal gland cell (tear secretion), Ceruminous gland cell in ear (wax secretion), Eccrine sweat gland dark cell (glycoprotein secretion), Eccrine sweat gland clear cell (small molecule secretion). Apocrine sweat gland cell (odoriferous secretion, sex -hormone sensitive), Gland of Moll cell in eyelid (specialized sweat gland), Sebaceous gland cell (lipid-rich sebum secretion), Bowman's gland cell in nose (washes olfactory epithelium), Brunner's gland cell in duodenum (enzymes and alkaline mucus), Seminal vesicle cell (secretes seminal fluid components, including fructose for swimming sperm), Prostate gland cell (secretes seminal fluid components), Bulbourethral gland cell (mucus secretion), Bartholin's gland cell (vaginal lubricant secretion), Gland of Littre cell (mucus secretion), Uterus endometrium cell (carbohydrate secretion), Isolated goblet cell of respiratory and digestive tracts (mucus secretion), Stomach lining mucous cell (mucus secretion), Gastric gland zymogenic cell (pepsinogen secretion), Gastric gland oxyntic cell (hydrochloric acid secretion), Pancreatic acinar cell (bicarbonate and digestive enzyme secretion), Paneth cell of small intestine (lysozyme secretion), Type II pneumocyte of lung (surfactant secretion), Clara cell of lung, Hormone secreting cells, Anterior pituitary cells, Somatotropes, Lactotropes, Thyrotropes, Gonadotropes, Corticotropes, Intermediate pituitary cell, Magnocellular neurosecretory cells, Gut and respiratory tract cells, Thyroid gland cells, thyroid epithelial cell, parafollicular cell, Parathyroid gland cells, Parathyroid chief cell, Oxyphil cell, Adrenal gland cells, chromaffin cells, Ley dig cell of testes, Theca interna cell of ovarian follicle, Corpus luteum cell of ruptured ovarian follicle, Granulosa lutein cells, Theca lutein cells, Juxtaglomerular cell (renin secretion), Macula densa cell of kidney, Metabolism and storage cells, Barrier function cells (Lung, Gut, Exocrine Glands and Urogenital Tract), Kidney, Type I pneumocyte (lining air space of lung), Pancreatic duct cell (centroacinar cell), Nonstriated duct cell (of sweat gland, salivary gland, mammary gland, etc.), Duct cell (of seminal vesicle, prostate gland, etc.), Epithelial cells lining closed internal body cavities, Ciliated cells with propulsive function, Extracellular matrix secretion cells, Contractile cells; Skeletal muscle cells, stem cell, Heart muscle cells, Blood and immune system cells, Erythrocyte (red blood cell), Megakaryocyte (platelet precursor), Monocyte, Connective tissue macrophage (various types), Epidermal Langerhans cell, Osteoclast (in bone), Dendritic cell (in lymphoid tissues), Microglial cell (in central nervous system), Neutrophil granulocyte, Eosinophil granulocyte, Basophil granulocyte, Mast cell, Helper T cell, Suppressor T cell, Cytotoxic T cell, Natural Killer T cell, B cell, Natural killer cell, Reticulocyte, Stem cells and committed progenitors for the blood and immune system (various types), Pluripotent stem cells, Totipotent stem cells, Induced pluripotent stem cells, adult stem cells, Sensory transducer cells, Autonomic neuron cells, Sense organ and peripheral neuron supporting cells, Central nervous system neurons and glial cells, Lens cells, Pigment cells, Melanocyte, Retinal pigmented epithelial cell, Germ cells, Oogonium/Oocyte, Spermatid, Spermatocyte, Spermatogonium cell (stem cell for spermatocyte), Spermatozoon, Nurse cells, Ovarian follicle cell, Sertoli cell (in testis), Thymus epithelial cell, Interstitial cells, and Interstitial kidney cells, or non-human cells that have been processed or irradiated, such as K562, NK92, etc. The target cells can be natural cells or modified cells.

Of particular interest are cancer cells. In some embodiments, the target cell is a cancer cell. Non-limiting examples of cancer cells include cells of cancers including Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia,Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloblastoma, Medulloepithelioma, Melanoma, Melanoma, Meningioma, Merkel Cell Carcinoma, Mesothelioma, Mesothelioma, Metastatic Squamous Neck Cancer with Occult Primary, Metastatic urothelial carcinoma, Mixed Mullerian tumor, Monocytic leukemia, Mouth Cancer, Mucinous tumor, Multiple Endocrine Neoplasia Syndrome, Multiple Myeloma, Multiple myeloma, Mycosis Fungoides, Mycosis fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neuroblastoma, Neurofibroma, Neuroma, Nodular melanoma, Non-Hodgkin Lymphoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral Cancer, Oral cancer, Oropharyngeal Cancer, Osteosarcoma, Osteosarcoma, Ovarian Cancer, Ovarian cancer, Ovarian Epithelial Cancer, Ovarian Germ Cell Tumor, Ovarian Low Malignant Potential Tumor, Paget's disease of the breast, Pancoast tumor, Pancreatic Cancer, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sezary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenstrom's macroglobulinemia, Warthin's tumor, Wilms' tumor, and combinations thereof. In some embodiments, the targeted cancer cell represents a subpopulation within a cancer cell population, such as a cancer stem cell. In some embodiments, the cancer is of a hematopoietic lineage, such as a lymphoma. The antigen can be a tumor associated antigen.

In some embodiments, the target cells form a tumor. A tumor treated with the methods herein can result in stabilized tumor growth (e.g., one or more tumors do not increase more than 1%, 5%, 10%, 15%, or 20% in size, and/or do not metastasize). In some embodiments, a tumor is stabilized for at least about 1, 2, 3, 4, 5, 6, 7, 8, 9, 10, 11, 12, or more weeks. In some embodiments, a tumor is stabilized for at least about 1, 2, 3, 4, 5, 6, 7, 8, 9, 10, 11, 12, or more months. In some embodiments, a tumor is stabilized for at least about 1, 2, 3, 4, 5, 6, 7, 8, 9, 10, or more years. In some embodiments, the size of a tumor or the number of tumor cells is reduced by at least about 5%, 10%, 15%, 20%, 25, 30%, 35%, 40%, 45%, 50%, 55%, 60%, 65%, 70%, 75%, 80%, 85%, 90%, 95% or more. In some embodiments, the tumor is completely eliminated, or reduced below a level of detection. In some embodiments, a subject remains tumor free (e.g. in remission) for at least about 1, 2, 3, 4, 5, 6, 7, 8, 9, 10, 11, 12, or more weeks following treatment. In some embodiments, a subject remains tumor free for at least about 1, 2, 3, 4, 5, 6, 7, 8, 9, 10, 11, 12, or more months following treatment. In some embodiments, a subject remains tumor free for at least about 1, 2, 3, 4, 5, 6, 7, 8, 9, 10, or more years after treatment.

Death of target cells can be determined by any suitable method, including, but not limited to, counting cells before and after treatment, or measuring the level of a marker associated with live or dead cells (e.g. live or dead target cells). Degree of cell death can be determined by any suitable method. In some embodiments, degree of cell death is determined with respect to a starting condition. For example, an individual can have a known starting amount of target cells, such as a starting cell mass of known size or circulating target cells at a known concentration. In such cases, degree of cell death can be expressed as a ratio of surviving cells after treatment to the starting cell population. In some embodiments, degree of cell death can be determined by a suitable cell death assay. A variety of cell death assays are available, and can utilize a variety of detection methodologies. Examples of detection methodologies include, without limitation, the use of cell staining, microscopy, flow cytometry, cell sorting, and combinations of these.

When a tumor is subject to surgical resection following completion of a therapeutic period, the efficacy of treatment in reducing tumor size can be determined by measuring the percentage of resected tissue that is necrotic (i.e., dead). In some embodiments, a treatment is therapeutically effective if the necrosis percentage of the resected tissue is greater than about 20% (e.g., at least about 30%, 40%, 50%, 60%, 70%, 80%, 90%, or 100%). In some embodiments, the necrosis percentage of the resected tissue is 100%, that is, no living tumor tissue is present or detectable.

Exposing a target cell to an immune cell or population of immune cells disclosed herein can be conducted either *in vitro* or *in vivo.* Exposing a target cell to an immune cell or population of immune cells generally refers to bringing the target cell in contact with the immune cell and/or in sufficient proximity such that an antigen of a target cell (e.g., membrane bound or non-membrane bound) can bind to the enhanced receptor or CAR or TCR expressed in the immune cell. Exposing a target cell to an immune cell or population of immune cells can also generally refer to bringing the target cell in contact with the immune cell and/or in sufficient proximity such that a target cell can bind to the non-specific killing immune cell. Exposing a target cell to an immune cell or population of immune cells *in vitro* can be accomplished by co-culturing the target cells and the immune cells. Target cells and immune cells can be co-cultured, for example, as adherent cells or alternatively in suspension. Target cells and immune cells can be co-cultured in various suitable types of cell culture media, for example with supplements, growth factors, ions, etc. Exposing a target cell to an immune cell or population of immune cells *in vivo* can be accomplished, in some cases, by administering the immune cells to a subject, for example a human subject, and allowing the immune cells to localize to the target cell via the circulatory system. In some cases, an immune cell can be delivered to the immediate area where a target cell is localized, for example, by direct injection.

Exposing can be performed for any suitable length of time, for example at least 1 minute, at least 5 minutes, at least 10 minutes, at least 30 minutes, at least 1 hour, at least 2 hours, at least 3 hours, at least 4 hours, at least 5 hours, at least 6 hours, at least 7 hours, at least 8 hours, at least 12 hours, at least 16 hours, at least 20 hours, at least 24 hours, at least 2 days, at least 3 days, at least 4 days, at least 5 days, at least 6 days, at least 1 week, at least 2 weeks, at least 3 weeks, at least 1 month or longer.

The various domains of enhanced receptor and CARs provided herein can be linked by means of chemical bond, e.g., an amide bond or a disulfide bond; a small, organic molecule (e.g., a hydrocarbon chain); an amino acid sequence such as a peptide linker (e.g., an amino acid sequence about 3-200 amino acids in length), or a combination of a small, organic molecule and peptide linker. Peptide linkers can provide desirable flexibility to permit the desired expression, activity and/or conformational positioning of the chimeric polypeptide. The peptide linker can be of any appropriate length to connect at least two domains of interest and is preferably designed to be sufficiently flexible so as to allow the proper folding and/or function and/or activity of one or both of the domains it connects. The peptide linker can have a length of at least 3, 5, 10, 15, 20, 25, 30, 35, 40, 45, 50, 55, 60, 65, 70, 75, 80, 85, 90, 95, or 100 amino acids. In some embodiments, a peptide linker has a length between about 0 and 200 amino acids, between about 10 and 190 amino acids, between about 20 and 180 amino acids, between about 30 and 170 amino acids, between about 40 and 160 amino acids, between about 50 and 150 amino acids, between about 60 and 140 amino acids, between about 70 and 130 amino acids, between about 80 and 120 amino acids, between about 90 and 110 amino acids. In some embodiments, the linker sequence can comprise an endogenous protein sequence. In some embodiments, the linker sequence comprises glycine, alanine and/or serine amino acid residues. In some embodiments, a linker can contain motifs, e.g., multiple or repeating motifs, of GS, GGS, GGGGS, GGSG, or SGGG. The linker sequence can include any naturally occurring amino acids, non-naturally occurring amino acids, or combinations thereof.

Any suitable delivery method can be used for introducing compositions and molecules (e.g., polypeptides and/or nucleic acid encoding polypeptides) of the disclosure into a host cell, such as an immune cell. The various components can be delivered simultaneously or temporally separated. The choice of method can be dependent on the type of cell being transformed and/or the circumstances under which the transformation is taking place (e.g., *in vitro, ex vivo,* or *in vivo*).

A method of delivery can involve contacting a target polynucleotide or introducing into a cell (or a population of cells such as immune cells) one or more nucleic acids comprising nucleotide sequences encoding the compositions of the disclosure. Suitable nucleic acids comprising nucleotide sequences encoding the compositions of the disclosure can include expression vectors, where an expression vector comprising a nucleotide sequence encoding one or more compositions of the disclosure is a recombinant expression vector.

Non-limiting examples of delivery methods or transformation include, for example, viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, and nanoparticle-mediated nucleic acid delivery.

In some aspects, the present disclosure provides methods comprising delivering one or more polynucleotides, or one or more vectors as described herein, or one or more transcripts thereof, and/or one or proteins transcribed therefrom, to a host cell. In some aspects, the disclosure further provides cells produced by such methods, and organisms (such as animals, plants, or fungi) comprising or produced from such cells.

Conventional viral and non-viral based gene transfer methods can be used to introduce nucleic acids in mammalian cells or target tissues. Such methods can be used to administer nucleic acids encoding compositions of the disclosure to cells in culture, or in a host organism. Non-viral vector delivery systems can include DNA plasmids, RNA (e.g. a transcript of a vector described herein), naked nucleic acid, and nucleic acid complexed with a delivery vehicle, such as a liposome. Viral vector delivery systems can include DNA and RNA viruses, which can have either episomal or integrated genomes after delivery to the cell.

Methods of non-viral delivery of nucleic acids can include lipofection, nucleofection, microinjection, biolistics, virosomes, liposomes, immunoliposomes, polycation or lipid:nucleic acid conjugates, naked DNA, artificial virions, and agent-enhanced uptake of DNA. Cationic and neutral lipids that are suitable for efficient receptor-recognition lipofection of polynucleotides can be used. Delivery can be to cells (e.g. in vitro or ex vivo administration) or target tissues (e.g. in vivo administration). The preparation of lipid:nucleic acid complexes, including targeted liposomes such as immunolipid complexes, can be used.

RNA or DNA viral based systems can be used to target specific cells in the body and trafficking the viral payload to the nucleus of the cell. Viral vectors can be administered directly (*in vivo*) or they can be used to treat cells in vitro, and the modified cells can optionally be administered (*ex vivo*). Viral based systems can include retroviral, lentivirus, adenoviral, adeno-associated and herpes simplex virus vectors for gene transfer. Integration in the host genome can occur with the retrovirus, lentivirus, and adeno-associated virus gene transfer methods, which can result in long term expression of the inserted transgene. High transduction efficiencies can be observed in many different cell types and target tissues.

Lentivirus can integrate its genome into host cells (such as 293 cells, or T cells). Lentivirus can use a three-plasmid system or a four-plasmid system.

The tropism of a retrovirus can be altered by incorporating foreign envelope proteins, expanding the potential target population of target cells. Lentiviral vectors are retroviral vectors that can transduce or infect non-dividing cells and produce high viral titers. Selection of a retroviral gene transfer system can depend on the target tissue. Retroviral vectors can comprise cis-acting long terminal repeats with packaging capacity for up to 6-10 kb of foreign sequence. The minimum cis-acting LTRs can be sufficient for replication and packaging of the vectors, which can be used to integrate the therapeutic gene into the target cell to provide permanent transgene expression. Retroviral vectors can include those based upon murine leukemia virus (MuLV), gibbon ape leukemia virus (GaLV), Simian Immuno deficiency virus (SIV), human immuno deficiency virus (HIV), and combinations thereof.

An adenoviral-based systems can be used. Adenoviral-based systems can lead to transient expression of the transgene. Adenoviral based vectors can have high transduction efficiency in cells and may not require cell division. High titer and levels of expression can be obtained with adenoviral based vectors. Adeno-associated virus ("AAV") vectors can be used to transduce cells with target nucleic acids, e.g., in the in vitro production of nucleic acids and peptides, and for in vivo and ex vivo gene therapy procedures.

Packaging cells can be used to form virus particles capable of infecting a host cell. Such cells can include 293 cells, (e.g., for packaging lentivirus or adenovirus), and Psi2 cells or PA317 cells (e.g., for packaging retrovirus). Viral vectors can be generated by producing a cell line that packages a nucleic acid vector into a viral particle. The vectors can contain the minimal viral sequences required for packaging and subsequent integration into a host. The vectors can contain other viral sequences being replaced by an expression cassette for the polynucleotide(s) to be expressed. The missing viral functions can be supplied in trans by the packaging cell line. For example, AAV vectors can comprise ITR sequences from the AAV genome which are required for packaging and integration into the host genome. Viral DNA can be packaged in a cell line, which can contain a helper plasmid encoding the other AAV genes, namely rep and cap, while lacking ITR sequences. The cell line can also be infected with adenovirus as a helper. The helper virus can promote replication of the AAV vector and expression of AAV genes from the helper plasmid. Contamination with adenovirus can be reduced by, e.g., heat treatment to which adenovirus is more sensitive than AAV. Additional methods for the delivery of nucleic acids to cells can be used, for example, as described in US20030087817, incorporated herein by reference.

A host cell can be transiently or non-transiently transfected with one or more vectors described herein. A cell can be transfected as it naturally occurs in a subject. A cell can be taken or derived from a subject and transfected. A cell can be derived from cells taken from a subject, such as a cell line. In some embodiments, a cell transfected with one or more vectors described herein is used to establish a new cell line comprising one or more vector-derived sequences. In some embodiments, a cell transiently transfected with the compositions of the disclosure (such as by transient transfection of one or more vectors, or transfection with RNA) is used to establish a new cell line comprising cells containing the modification but lacking any other exogenous sequence.

Any suitable vector compatible with the host cell can be used with the methods of the disclosure. Non-limiting examples of vectors for eukaryotic host cells include pXT1, pSG5 (StratageneTM), pSVK3, pBPV, pMSG, and pSVLSV40 (PharmaciaTM).

Contacting the cells with a composition of the can occur in any culture media and under any culture conditions that promote the survival of the cells. For example, cells may be suspended in any appropriate nutrient medium that is convenient, such as Iscove's modified DMEM or RPMI 1640, supplemented with fetal calf serum or heat inactivated goat serum (about 5-10%), L-glutamine, a thiol, particularly 2-mercaptoethanol, and antibiotics, e.g. penicillin and streptomycin. The culture may contain growth factors to which the cells are responsive. Growth factors, as defined herein, are molecules capable of promoting survival, growth and/or differentiation of cells, either in culture or in the intact tissue, through specific effects on a transmembrane receptor. Growth factors can include polypeptides and non-polypeptide factors.

In numerous embodiments, the chosen delivery system is targeted to specific tissue or cell types. In some cases, tissue- or cell- targeting of the delivery system is achieved by binding the delivery system to tissue- or cell-specific markers, such as cell surface proteins. Viral and non-viral delivery systems can be customized to target tissue or cell-types of interest.

Pharmaceutical compositions containing molecules (e.g., polypeptides and/or nucleic acids encoding polypeptides) or immune cells described herein can be administered for prophylactic and/or therapeutic treatments. In therapeutic applications, the compositions can be administered to a subject already suffering from a disease or condition, in an amount sufficient to cure or at least partially arrest the symptoms of the disease or condition, or to cure, heal, improve, or ameliorate the condition. Amounts effective for this use can vary based on the severity and course of the disease or condition, previous therapy, the subject's health status, weight, and response to the drugs, and the judgment of the treating physician.

Multiple therapeutic agents can be administered in any order or simultaneously. If simultaneously, the multiple therapeutic agents can be provided in a single, unified form, or in multiple forms, for example, as multiple separate pills. The molecules or cell solutions can be packed together or separately, in a single package or in a plurality of packages. One or all of the therapeutic agents can be given in multiple doses. If not simultaneous, the timing between the multiple doses may vary to as much as about 1 month to 24 months.

Molecules and cells described herein can be administered before, during, or after the occurrence of a disease or condition, and the timing of administering the composition containing a compound can vary. For example, the pharmaceutical compositions can be used as a prophylactic and can be administered continuously to subjects with a propensity to conditions or diseases in order to prevent the occurrence of the disease or condition. The molecules, cells and pharmaceutical compositions can be administered to a subject during or as soon as possible after the onset of the symptoms. The administration of the molecules can be initiated within the first 48 hours of the onset of the symptoms, within the first 24 hours of the onset of the symptoms, within the first 6 hours of the onset of the symptoms, or within 3 hours of the onset of the symptoms. The initial administration can be via any route practical, such as by any route described herein using any formulation described herein. A molecule can be administered as soon as is practicable after the onset of a disease or condition is detected or suspected, and for a length of time necessary for the treatment of the disease, such as, for example, from about 1 month to about 3 months. The length of treatment can vary for each subject.

A molecule can be packaged into a biological compartment. A biological compartment comprising the molecule can be administered to a subject. Biological compartments can include, but are not limited to, viruses (lentivirus, adenovirus), nanospheres, liposomes, quantum dots, nanoparticles, microparticles, nanocapsules, vesicles, polyethylene glycol particles, hydrogels, and micelles.

For example, a biological compartment can comprise a liposome. A liposome can be a self-assembling structure comprising one or more lipid bilayers, each of which can comprise two monolayers containing oppositely oriented amphipathic lipid molecules. Amphipathic lipids can comprise a polar (hydrophilic) headgroup covalently linked to one or two or more non-polar (hydrophobic) acyl or alkyl chains. Energetically unfavorable contacts between the hydrophobic acyl chains and a surrounding aqueous medium induce amphipathic lipid molecules to arrange themselves such that polar headgroups can be oriented towards the bilayer's surface and acyl chains are oriented towards the interior of the bilayer, effectively shielding the acyl chains from contact with the aqueous environment.

Examples of preferred amphipathic compounds used in liposomes can include phosphoglycerides and sphingolipids, representative examples of which include phosphatidylcholine, phosphatidylethanolamine, phosphatidylserine, phosphatidylinositol, phosphatidic acid, phoasphatidylglycerol, palmitoyloleoyl phosphatidylcholine, lysophosphatidylcholine, lysophosphatidylethanolamine, dimyristoylphosphatidylcholine (DMPC), dipalmitoylphosphatidylcholine (DPPC), dioleoylphosphatidylcholine, distearoylphosphatidylcholine (DSPC), dilinoleoylphosphatidylcholine and egg sphingomyelin, or any combination thereof.

A biological compartment can comprise a nanoparticle. A nanoparticle can comprise a diameter of from about 40 nanometers to about 1 .5 micrometers, from about 50 nanometers to about 1.2 micrometers, from about 60 nanometers to about 1 micrometer, from about 70 nanometers to about 800 nanometers, from about 80 nanometers to about 600 nanometers, from about 90 nanometers to about 400 nanometers, from about 100 nanometers to about 200 nanometers.

In some cases, as the size of the nanoparticle increases, the release rate can be slowed or prolonged and as the size of the nanoparticle decreases, the release rate can be increased.

The amount of albumin in the nanoparticles can range from about 5% to about 85% albumin (v/v), from about 10% to about 80%, from about 15% to about 80%, from about 20% to about 70% albumin (v/v), from about 25% to about 60%, from about 30% to about 50%, or from about 35% to about 40%. The pharmaceutical composition can comprise up to 30, 40, 50, 60, 70 or 80% or more of the nanoparticle. In some cases, the nucleic acid molecules of the disclosure can be bound to the surface of the nanoparticle.

A biological compartment can comprise a virus. The virus can be a delivery system for the pharmaceutical compositions of the disclosure. Exemplary viruses can include lentivirus, retrovirus, adenovirus, herpes simplex virus I or II, parvovirus, reticuloendotheliosis virus, and adeno-associated virus (AAV). Pharmaceutical compositions of the disclosure can be delivered to a cell using a virus. The virus can infect and transduce the cell *in vivo*, *ex vivo*, or *in vitro.* In *ex vivo* and *in vitro* delivery, the transduced cells can be administered to a subject in need of therapy.

Pharmaceutical compositions can be packaged into viral delivery systems. For example, the compositions can be packaged into virions by a HSV-1 helper virus-free packaging system.

Viral delivery systems (e.g., viruses comprising the pharmaceutical compositions of the disclosure) can be administered by direct injection, stereotaxic injection, intracerebroventricularly, by minipump infusion systems, by convection, catheters, intravenous, parenteral, intraperitoneal, and/or subcutaneous injection, to a cell, tissue, or organ of a subject in need. In some cases, cells can be transduced *in vitro* or *ex vivo* with viral delivery systems. The transduced cells can be administered to a subject having a disease. For example, a stem cell can be transduced with a viral delivery system comprising a pharmaceutical composition and the stem cell can be implanted in the patient to treat a disease.

In some cases, the dose of cells given to a subject can be less than 1×10⁴ cells/kg, or about 1×10⁴ cells/kg, about 2×10⁴ cells/kg, about 3×10⁴ cells/kg, about 4×10⁴ cells/kg, about 5×10⁴ cells/kg, about 6×10⁴ cells/kg, about 7×10⁴ cells/kg, about 8×10⁴ cells/kg, about 9×10⁴ cells, about 1×10⁵ cells/kg, about 2×10⁵ cells/kg, about 3×10⁵ cells/kg, about 4×10⁵ cells/kg, about 5×10⁵ cells/kg, about 6×10⁵ cells/kg, about 7×10⁵ cells/kg, about 8×10⁵ cells/kg, about 9×10⁵ cells, about 1×10⁶ cells/kg, about 2×10⁶ cells/kg, about 3×10⁶ cells/kg, about 4×10⁶ cells/kg, about 5×10⁶ cells/kg, about 6×10⁶ cells/kg, about 7×10⁶ cells/kg, about 8×10⁶ cells/kg, about 9×10⁶ cells, about 1×10⁷ cells/kg, about 5×10⁷ cells/kg, about 1×10⁸ cells/kg or more. The cell dose herein can be calculated based on the number of effectively modified cells successfully transfected, or can be calculated based on the total number of cells.

Introduction of the biological compartments into cells can occur by viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro-injection, nanoparticle-mediated nucleic acid delivery, and the like.

In some embodiments, immune cells expressing a subject system are administered. Immune cells expressing a subject system can be administered before, during, or after the occurrence of a disease or condition, and the timing of administering the immune cells can vary. For example, immune cells expressing a subject system can be used as a prophylactic and can be administered continuously to subjects with a propensity to conditions or diseases in order to prevent the occurrence of the disease or condition. The immune cells can be administered to a subject during or as soon as possible after the onset of the symptoms. The administration can be initiated within the first 48 hours of the onset of the symptoms, within the first 24 hours of the onset of the symptoms, within the first 6 hours of the onset of the symptoms, or within 3 hours of the onset of the symptoms. The initial administration can be via any suitable route, such as by any route described herein using any formulation described herein. Immune cells can be administered as soon as is practicable after the onset of a disease or condition is detected or suspected, and for a length of time necessary for the treatment of the disease, such as, for example, from about 1 month to about 3 months. The length of treatment can vary for each subject.

A molecule described herein (e.g., polypeptide and/or nucleic acid) can be present in a composition in a range of from about 1 mg to about 2000 mg; from about 5 mg to about 1000 mg, from about 10 mg to about 25 mg to 500 mg, from about 50 mg to about 250 mg, from about 100 mg to about 200 mg, from about 1 mg to about 50 mg, from about 1 mg to about 50 mg, from about 50 mg to about 100 mg, from about 100 mg to about 150 mg, from about 150 mg to about 200 mg, from about 200 mg to about 250 mg, from about 250 mg to about 300 mg, from about 300 mg to about 350 mg, from about 350 mg to about 400 mg, from about 400 mg to about 450 mg, from about 450 mg to about 500 mg, from about 500 mg to about 550 mg, from about 550 mg to about 600 mg, from about 600 mg to about 650 mg, from about 650 mg to about 700 mg, from about 700 mg to about 750 mg, from about 750 mg to about 800 mg, from about 800 mg to about 850 mg, from about 850 mg to about 900 mg, from about 900 mg to about 950 mg, or from about 950 mg to about 1000 mg.

A molecule (e.g., polypeptide and/or nucleic acid) described herein can be present in a composition in an amount of about 1 mg, about 2 mg, about 3 mg, about 4 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 125 mg, about 150 mg, about 175 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, or about 2000 mg.

A molecule (e.g., polypeptide and/or nucleic acid) described herein can be present in a composition that provides at least 0.1, 0.5, 1, 1.5, 2, 2.5 3, 3.5, 4, 4.5, 5, 5.5, 6, 6.5, 10 or more units of activity/mg molecule. The activity can be regulation of gene expression. In some embodiments, the total number of units of activity of the molecule delivered to a subject is at least 25,000, 30,000, 35,000, 40,000, 45,000, 50,000, 60,000, 70,000, 80,000, 90,000, 110,000, 120,000, 130,000, 140,000, 150,000, 160,000, 170,000, 180,000, 190,000, 200,000, 210,000, 220,000, 230,000, or 250,000 or more units. In some embodiments, the total number of units of activity of the molecule delivered to a subject is at most 25,000, 30,000, 35,000, 40,000, 45,000, 50,000, 60,000, 70,000, 80,000, 90,000, 110,000, 120,000, 130,000, 140,000, 150,000, 160,000, 170,000, 180,000, 190,000, 200,000, 210,000, 220,000, 230,000, or 250,000 or more units.

### EXAMPLES

In order to more comprehensively understand and utilize the present invention, the present invention will be described in detail below with reference to examples and drawings. The examples are only intended to illustrate the present invention, without any intention of limiting the scope of the present invention. The scope of the present invention is specifically defined by the appended claims.

### Example 1. Preparation of CAR-T Cells and Super TIL Cells from Peripheral Blood

In Examples 1-7, we used CD19 as the booster antigen, and CD19-targeting CAR (for the sequence of the CAR being used, see SEQ ID NO: 1 of Chinese Invention Application Publication No. CN 110016465 A) as aBA-CAR.

Human CAR-T cells were prepared as follows: after collecting human PBMC cells, T cells in CD19-cells derived from PBMC were activated by using CD3/CD28 magnetic beads and transfected with CD19-CAR lentivirus.

Human super TIL cells were prepared as follows: after collecting monocytes from patients with advanced tumors, magnetic beads were used to capture PD1+ T cells. Lentiviral vectors containing the following two coding sequences were constructed: the nucleotide sequence encoding the CD19 CAR as aBA-CAR, and the nucleotide sequence encoding the enhanced receptor-PD1-CD28. The constructed lentivirus was used to transfect the captured PD1+ T cells.

### Example 2. Booster adjuvant - autologous B cells boost expansion of super TILs

B cells were isolated from the apheresis cells of patients and cultured to the order of 10⁷ cells. B cells naturally carry CD19 membrane antigen as booster antigen.

The super TIL cells prepared as in Example 1 were divided into two groups, A and B, each having the order of 10⁶ cells, in which group A was subjected to natural growth as a control, and group B was treated as follows. The 10⁶-order autologous B cells were co-cultured with 10⁶-order super TIL cells, 10 days for each round, adding 10⁶-order autologous B cells to the system in each round, and for 3 consecutive rounds. It could be observed that super TILs of each round has increased by more than 5 times as compared with the previous round, while the number of super TILs in the control group, group A, did not significantly increase.

### Example 3. Booster adjuvant 2 - autologous CD19+ T cells boost expansion of super TILs

T cells were isolated from the apheresis cells of patients, and the isolated T cells were transfected by the lentivirus carrying CD19 coding sequence to obtain CD19+ T cells as booster cells, which were cultured to the order of 10⁷ cells by stimulation with CD3/CD28 magnetic beads.

The super TIL cells were divided into two groups, A and B, each having the order of 10⁶ cells, in which group A was subjected to natural growth as a control, and group B was treated as follows. The 10⁶-order autologous CD19+ T cells were co-cultured with 10⁶-order super TIL cells, 10 days for each round, adding 10⁶-order autologous CD19+ T cells to the system in each round, and for 3 consecutive rounds. It could be observed that super TILs of each round has increased by more than 5 times as compared with the previous round, while the number of super TILs in the control group, group A, did not significantly increase.

### Example 4. Booster adjuvant 3 - patient autologous NK cells boost expansion of super TILs

NK cells were isolated from the apheresis cells of patients, and the isolated NK cells were transfected by the lentivirus carrying CD19 coding sequence to obtain CD19+ NK cells as booster cells, which were cultured to the order of 10⁷ cells by stimulation with CD3/CD28 magnetic beads.

The super TIL cells were divided into two groups, A and B, each having the order of 10⁶ cells, in which group A was subjected to natural growth as a control, and group B was treated as follows. The 10⁶-order autologous CD19+ NK cells were co-cultured with 10⁶-order super TIL cells, 10 days for each round, adding 10⁶-order autologous CD19+ NK cells to the system in each round, and for 3 consecutive rounds. It could be observed that super TILs of each round has increased by more than 5 times as compared with the previous round, while the number of super TILs in the control group, group A, did not significantly increase.

### Example 5. Booster adjuvant 4 - heterologous B cells boost expansion of super TILs

B cells from another individual volunteer who was different from the subject was cultured to a number of the order of 10⁷. B cells naturally carry CD19 membrane antigen as booster antigen.

The super TIL cells were divided into two groups, A and B, each having the order of 10⁶ cells, in which group A was subjected to natural growth as a control, and group B was treated as follows. The 10⁶-order heterologous B cells were co-cultured with 10⁶-order super TIL cells, 10 days for each round, adding 10⁶-order heterologous B cells to the system in each round, and for 3 consecutive rounds. It could be observed that super TILs of each round has increased by more than 5 times as compared with the previous round, while the number of super TILs in the control group, group A, did not significantly increase.

### Example 6. Booster adjuvant 5 - heterologous NK cells boost expansion of super TILs

NK cells were isolated from the apheresis cells of another individual (heterologous) volunteer different from the subject, and the isolated heterologous NK cells were transfected by the lentivirus carrying CD19 coding sequence to obtain heterologous CD19+ NK cells as booster cells, which were cultured to the order of 10⁷.

The super TIL cells were divided into two groups, A and B, each having the order of 10⁶ cells, in which group A was subjected to natural growth as a control, and group B was treated as follows. The 10⁶-order heterologous CD19+ NK cells were co-cultured with 10⁶-order super TIL cells, 10 days for each round, adding 10⁶-order heterologous CD19+ NK cells to the system in each round, and for 3 consecutive rounds. It could be observed that super TILs of each round has increased by more than 5 times as compared with the previous round, while the number of super TILs in the control group, group A, did not significantly increase.

### Example 7. Booster adjuvant 6 - nanoparticles carrying booster antigen boost expansion of super TILs

CD19 protein was attached to nanoparticles to construct nanoparticles carrying the booster antigen.

The super TIL cells were divided into two groups, A and B, each having the order of 10⁶ cells, in which group A was subjected to natural growth as a control, and group B was treated as follows. The nanoparticles with a total of about 1 microgram of CD19 protein loaded were co-cultured with 10⁶-order super TIL cells, 7 days for each round, adding nanoparticles with a total of about 1 microgram of CD19 protein loaded to the system in each round, and for 3 consecutive rounds. It could be observed that super TILs of each round has increased by more than 5 times as compared with the previous round, while the number of super TILs in the control group, group A, did not significantly increase.

### Example 8. Two-grade boosting system of autologous T cells boosts expansion of super TILs

Two types of booster cells were constructed with patient's own T cells, constructing a two-grade boosting system.

First grade boosting cells: T cells were transfected with a lentivirus loaded with EGFRviii coding sequence, resulting in EGFRviii+ T cells as booster cells, and the cells were cultured to the order of 10⁷ with stimulation by CD3/CD28 magnetic beads.

Second grade boosting cells: T cells were transfected with a lentivirus loaded with both the CD19 coding sequence and the coding sequence of EGFRviii-targeting CAR (the coding nucleotide sequence is shown in SEQ ID NO: 1), resulting in CD19+ EGFRviii+-CAR-T cells, and the cells were cultured to the order of 10⁷ with stimulation by CD3/CD28 magnetic beads.

Super TIL cells were divided into three groups, A, B, and C, each having the order of 10⁶ cells, in which group A was subjected to natural growth as a control, group B and group C were treated as follows.

In group B, 10⁶-order of the second grade booster cells (^{C}D19⁺ EGFRviii-CAR-T cells) were co-cultured with super TILs, as positive control for using single grade booster cells. That is, 10⁶-order of the second grade booster cells were co-cultured with 10⁶-order of super TILs, 10 days for each round, adding 10⁶-order second grade booster cells to the system in each round, and for 3 consecutive rounds.

In group C, 10⁶-order of the first grade booster cells (EGFRviii⁺ T cells) were co-cultured with the second grade booster cells (^{C}D19⁺ EGFRviii-CAR-T cells) for 10 days, followed by addition of 10⁶-order of super TIL cells, and simultaneous addition of 10⁶-order of the first grade booster cells; super TILs were co-cultured under this condition, 10 days for each round, adding 10⁶-order first grade booster cells every 10 days, and for 3 consecutive rounds.

It could be observed that super TILs of each round has increased by more than 5 times as compared with the previous round in group B, while group C had more multiples of amplification in each round than group B. The number of super TILs in group A did not significantly increase.

### Example 9. Two-grade boosting system of heterologous T cells boosts expansion of super TILs

Two types of booster cells were constructed with heterologous NK cells and patient's own T cells, constructing a two-grade boosting system.

First grade boosting cells: heterologous NK cells were transfected with a lentivirus loaded with EGFRviii coding sequence, resulting in EGFRviii+ NK cells as booster cells, and the cells were cultured to the order of 10⁷ with stimulation by CD3/CD28 magnetic beads.

Second grade boosting cells: autologous T cells were transfected with a lentivirus loaded with both the CD19 coding sequence and the coding sequence of EGFRviii-targeting CAR (SEQ ID NO: 1), resulting in CD19+ EGFRviii+-CAR-T cells, and the cells were cultured to the order of 10⁷ with stimulation by CD3/CD28 magnetic beads.

Super TIL cells were divided into three groups, A, B, and C, each having the order of 10⁶ cells, in which group A was subjected to natural growth as a control.

Group B was treated as follows: 10⁶-order of the second grade booster cells (CD19⁺ EGFRviii-CAR-T cells) were co-cultured with super TILs, as positive control for using single grade booster cells. That is, 10⁶-order of the second grade booster cells were co-cultured with 10⁶-order of super TILs, 10 days for each round, adding 10⁶-order second grade booster cells to the system in each round, and for 3 consecutive rounds

Group C was treated as follows: 10⁶-order of the first grade booster cells (EGFRviii⁺ NK cells) were co-cultured with the second grade booster cells (CD19⁺ EGFRviii-CAR-T cells) for 10 days, followed by addition of 10⁶-order of super TIL cells, and simultaneous addition of 10⁶-order of the first grade booster cells; super TILs were co-cultured under this condition, 10 days for each round, adding 10⁶-order first grade booster cells every 10 days, and for 3 consecutive rounds.

It could be observed that super TILs of each round has increased by more than 5 times as compared with the previous round in group B, while group C had more multiples of amplification in each round than group B. The number of super TILs in group A did not significantly increase.

### Example 10. Comparison of the effects of B cells and two artificial booster cells with CD19-CD86 as booster antigen in boosting expansion of CAR-T cells

This example compares the booster expansion effects of three booster cells on specific immune cells-CAR-T cells targeting CD19, in which the three booster cells are: 1) natural B cells expressing CD19; 2) artificial booster cells with a combination of CD19 and CD86 as the booster antigen and T cell as the booster cell (abbreviated as T/CD19-86); and 3) artificial booster cells with a combination of CD19 and CD86 as the booster antigen and K562 cell as the booster cell (abbreviated as K562/CD19-86).

The experiment protocol of this example is as follows.
**1. Preparation of PBMC and B cells**
   1) PBMCs were isolated and purified from fresh bloods;
   2) CD19+ cells (B cells) and CD19- cells were isolated by cell sorting.
**2. Preparation of CAR-T cells targeting CD19 (referred as CART19)**
   1) 293T cells were transfected to prepare a lentivirus comprising CD19-CAR cDNA;
   2) T cells in the CD19-cells derived from PBMC were stimulated by CD3/CD28 magnetic beads;
   3) T cells in step 2) were transfected by the lentivirus loaded with CD19-CAR;
   4) Expression of CD19-CAR on the transfected T cells was analyzed.
**3. Preparation of booster cell T/CD19-86**
   1) 293T cells were transfected to prepare a lentivirus comprising CD19-CD86 cDNA;
   2) T cells in the CD19-cells derived from PBMC were stimulated by CD3/CD28 magnetic beads;
   3) T cells in step 2) were transfected by the lentivirus loaded with CD19-CD86;
   4) Expression of CD19-CD86 on the transfected T cells was analyzed.
**4. Preparation of booster cell K562/CD19-86**
   1) 293T cells were transfected to prepare a lentivirus comprising CD19-CD86 cDNA;
   2) K562 cells were transfected by the lentivirus loaded with CD19-CD86;
   3) Expression of CD19-CD86 was analyzed and CD19+CD86+ cells were sorted;
   4) Conduct expanded culture, 100 Gray γ-ray irradiation, aliquoted, and frozen.
**5. Co-culture of CART19 with three booster cells, respectively**
   1) CART19 were divided into three equal parts, and each was mixed with one of the three booster cells as prepared in above 2-4, namely B cells, T /CD19-CD86, K562/CD19-CD86 blends, with the ratio of the CART19 cells and booster cells being 1:1;
   2) The cell concentration was adjusted to 1 × 10⁶/ml, and 2 U/ml Tscm and 100 U/ml IL-2 were supplemented into the medium (same below);
   3) Cells were cultured in an incubator, with half of the medium being exchanged every Monday and Wednesday (reserve half the volume of the old medium after exchanging the medium, add half the volume of fresh medium, and maintain the cell concentration at 1 × 10⁶/ml); flow cytometry analysis was conducted on Friday, the booster cells were calculated and added according to the proportion of CART19 cells to maintain the ratio of CART19 cells to booster cells at 1:1, and all the medium was exchanged with fresh medium;
   4) After adding booster cells for the third time, culture was continued for a week, and the proportion of CART19 cells was analyzed by flow cytometry.
**6. Data analysis**

The expansion factor of CART19 after co-culture of the three booster cells and CART19 was calculated, respectively, and the results are as follows:
B cell group, CART19 was expanded 487 times;
T/CD19-CD86 group, CART19 was expanded 134 times;
K562/CD19-CD86 group, CART19 was expanded 2719 times.

### Example 11. Comparison of the effects of artificial booster cells with artificial booster cells comprising a composition of EGFRviii as booster antigen in boosting expansion of two CAR-Ts

This example compares the boosting expansion effect of artificial booster cells expressing a composition containing EGFRviii on two specific immune cells, one of which comprises an enhanced receptor. Among them, the composition comprising EGFRviii is a composition of EGFRviiit (truncated EGFRviii with the intracellular segment removed), CD86, CD137L, and mbIL15-IL15Ra, the artificial booster cell is K562, and the cell expressing the composition is indicated as K562/EGFRviiit. The two immune cells being boosted are: 1) CAR-T cells targeting EGFRviii (expressed as EGFRviii-CAR-T); and 2) CAR-T cells targeting EGFRviii and additionally modified with PD1-CD28 enhanced receptor (expressed asEGFRviii-ECAR-T).

The experiment protocol of this example is as follows.
**1. Preparation of PBMC**
   1) PBMCs were isolated and purified from fresh bloods;
   2) aliquoted and frozen.
**2. Preparation of EGFRviii-CART and EGFRviii-ECART**
   1) 293T cells were transfected to prepare lentivirus comprising EGFRviii-CAR and EGFRviii-CAR/PD1-CD28;
   2) PBMCs were resuscitated, and T cells were stimulated with CD3/CD28 magnetic beads;
   3) The PBMCs treated with CD3/CD28 magnetic bead were transfected with the three lentiviruses as prepared, respectively;
   4) The expression of EGFRvIII-CAR in T cells was analyzed.
**3. Preparation of booster cell K562/EGFRviiit**
   1) 293T cells were transfected to prepare a lentivirus comprising a composition of EGFRviiit;
   2) K562 cells were transfected by the lentivirus;
   3) Expression of EGFRviiit, CD86, CD137L and mbIL15-IL15Ra was analyzed and EGFRviiit+CD86+CD137L+mbIL15+ cells were sorted;
   4) Conduct expanded culture, 100 Gray γ-ray irradiation, aliquoted, and frozen.
**4. Co-culture of two CAR-Ts with booster cell K562/EGFRviiit**
   1) EGFRviii-CART and EGFRviii-ECART, respectively, were mixed with above described booster cell K562/EGFRviiit, wherein the ratio of the CAR+ T cells and the booster cells is 1:1;
   2) The cell concentration was adjusted to 1 × 10⁶/ml, and 30 ng/ml IL-21, 50 U/ml IL-2, 10 ng/ml IL-15, 50 ng/ml IL-7 were supplemented into the medium (same below);
   3) Cells were cultured in an incubator, with half of the medium being exchanged every Monday and Wednesday (reserve half the volume of the old medium after exchanging the medium, add half the volume of fresh medium, and maintain the cell concentration at 1 × 10⁶/ml); flow cytometry analysis was conducted on Friday, the booster cells were calculated and added according to the proportion of CAR+ T cells to maintain the ratio of CAR+ T cells to booster cells at 1:1, and all the medium was exchanged with fresh medium;
   5) After adding booster cells for the fourth time, culture was continued for a week, and the proportion of CAR+ T cells was analyzed by flow cytometry.
**5. Data analysis**

The expansion factor of two CAR-Ts after co-culture of the booster cell K562/EGFRviiit with EGFRviii-CART and with EGFRviii-ECART, respectively, for three weeks was calculated, and the results are as follows:
EGFRviii-CART group, the number of CAR-T cells were expanded 3.6 times;
EGFRviii-ECARTgroup, the number of CAR-T cells were expanded 21.8 times.

## Claims

1. An adjuvant for assisting immune cell therapy, **characterized in that** the adjuvant comprises a booster antigen (BA), said booster antigen being capable of activating certain modified therapeutic immune cells *in vivo,* and boosting the number of the immune cells to expand *in vivo.*

2. The adjuvant according to claim 1, **characterized in that** the booster antigen is a marker or membrane protein on the surface of peripheral blood cells or tumor cells or pathogens, wherein the peripheral blood cells are selected from B cells, T cells, and NK cells , granulocytes, red blood cells, platelets, the pathogen is selected from HPV virus, EB virus, Helicobacter pylori (HP), hepatitis B virus (HBV), HIV (HIV).

3. The adjuvant according to claim 1, **characterized in that** the booster antigen is a wild type or variant of complete or partial fragment of a human natural protein, or a wild type or variant of complete or partial fragment of a plurality of human natural proteins.

4. The adjuvant according to claim 1, **characterized in that** the booster antigen is CD19 or EGFRviii, or a composition comprising CD19 and/or CD86 and/or CD137L, or a composition containing EGFRviii and/or CD86 and/or CD137L.

5. The adjuvant according to any one of claims 1 to 4, **characterized in that** the adjuvant is selected from protein, compound, complex, booster cell or artificial nanomaterial.

6. The adjuvant according to claim 5, **characterized in that** the booster cells are one or more of peripheral blood mononuclear cells (PBMC) or B cells, T cells, and NK cells derived from a subject's autologous or heterologous body.

7. The adjuvant according to claim 6, **characterized in that** the booster cells are unmodified natural cells, or modified cells, or immortalized B cells or NK cells (such as NK92) or K562 cells.

8. The adjuvant according to claim 5, **characterized in that** the adjuvant is booster cell, wherein said booster antigen is transmembrane expressed on the booster cell, said booster antigen is a fusion protein composed of an extracellular binding domain and a transmembrane region, wherein the therapeutic immune cell can recognize the extracellular binding domain, the transmembrane region is part or complete structure of a protein naturally expressed by the booster cell.

9. The adjuvant according to claim 8, **characterized in that** the booster cells are booster T cells, the extracellular binding domain is a partial region of EGFRviii or CD19, and the transmembrane region is a partial region of CD3.

10. A chimeric antigen receptor (aBA-CAR) capable of specifically binding to the booster antigen according to any one of claims 1 to 4 or 6 to 9, **characterized in that** the aBA-CAR comprises an antigen binding domain of an antibody specifically binding to the booster antigen.

11. The aBA-CAR according to claim 10, comprising CD3ζ, a variable region of an antibody targeting the booster antigen, and a co-stimulatory molecule.

12. The aBA-CAR according to claim 10, **characterized in that** the co-stimulatory molecule is selected from one or more of CD28, 41BBz and ICOS.

13. The aBA-CAR according to any one of claims 10 to 12, comprising a switch element that regulates the expression of CAR or CAR-T cells, or makes CAR-T or CAR-NK cells committed to suicide or apoptosis, such as TET-ON.

14. A therapeutic immune cell, **characterized in that** the immune cell has dual recognition specificity, wherein the dual recognition specificity refers to the specific recognition of target cell and the specific recognition of the booster antigen comprised in the adjuvant according to claim 1, wherein the target cells are cells harmful to human body, such as tumor cells, or pathogen cells such as viruses, bacteria, fungus, such that the immune cells can be activated and expanded *in vivo* by the adjuvant according to claim 1, wherein the target of target cell recognized by the immune cell and the target of the booster antigen are different or the same.

15. The therapeutic immune cell according to claim 14, **characterized in that** the immune cell expresses the aBA-CAR according to claim 10.

16. The therapeutic immune cell according to claim 14 or claim 15, **characterized in that** the immune cell is any one of T cell or NK cell, or a mixture thereof.

17. The therapeutic immune cell according to claim 16, **characterized in that** the immune cell is T cell.

18. The therapeutic immune cell according to claim 17, **characterized in that** the T cell is tumor-recognizing T cells or tumor antigen-reactive T cells, which recognizes tumor or tumor antigen by means of unmodified natural TCRs to recognize tumor neoantigens, or tumor-associated antigens (TAA), or cancer testis antigens, or viral antigens (such as viral antigens on the surface of cells infected by HPV or EBV and become cancerous), wherein the tumor-recognizing T cell or tumor antigen-reactive T cell can be obtained by vaccine induction, or can be obtained from tumor infiltrating lymphocytes (TIL), or can be obtained by positive or negative screening with certain markers of peripheral blood (such as PD1, TIM3, CD137, CD39, CD28).

19. The therapeutic immune cell according to claim 17, **characterized in that** the T cells are derived from tumor-infiltrating T lymphocytes (TIL), or TIL that has been subjected to positive or negative screen with one or more of markers such as PD1, TIM3, CD137, CD39, CD28.

20. The therapeutic immune cell according to claim 17, **characterized in that** the T cells are derived from peripheral blood mononuclear cells (PBMC), or derived from PBMC that has been subjected to positive or negative screen with one or more of markers such as PD1, TIM3, CD137, and CD39

21. The therapeutic immune cell according to any one of claims 17 to 20, **characterized in that** the T cell is subjected to additional gene modification other than aBA-CAR modification.

22. The therapeutic immune cell according to any one of claims 17 to 21, **characterized in that** the T cell is CAR-T or TCR-T cell modified to express CAR or exogenous TCR in addition to aBA-CAR modification, and the CAR or exogenous TCR confers specificity of recognizing target cells other than its natural TCR.

23. The therapeutic immune cell according to any one of claims 17 to 22, **characterized in that** the T cell expresses an enhanced receptor, wherein the enhanced receptor is a transmembrane protein and is composed of extracellular domain (ECD) and intracellular domain of T cell, the ECD is the complete sequence structure of any one of a receptor, ligand, antibody of the membrane protein of the target cell of the immune cell, or other protein structure that can bind to the membrane protein, or a part comprising a binding domain thereof, the ICD is any one of co-stimulatory signal proteins such as CD28 or 41BB or ICOS, and does not comprise CD3.

24. The therapeutic immune cell according to claim 23, **characterized in that** the ECD of the enhanced receptor generates an inhibitory signal on the T cells upon binding to its ligand, while an intact enhanced receptor generates an activating signal on the T cells upon binding to its ligand.

25. The therapeutic immune cell according to claim 16, **characterized in that** the immune cell is NK cell that has subjected to additional gene modification other than the modification to express aBA-CAR.

26. The therapeutic immune cell according to claim 25, **characterized in that** the NK cell is CAR-NK cell modified to express CAR in addition to aBA-CAR modification, and the CAR enables the NK cell to recognize and kill the target cell.

27. The therapeutic immune cell according to claim 26, **characterized in that** the cell is modified to comprise a partial structure of NKG2D.

28. The adjuvant according to claim 1, **characterized in that** the adjuvant is a cascade booster system composed of n kinds of booster cells according to claim 9, wherein n is a positive integer, wherein the first-grade booster cells express the first booster antigen (BA1), the second-grade booster cells express the second booster antigen (BA2) and aBA1-CAR specifically targeting the first booster antigen, and the third-grade booster cells express the third booster antigen (BA3) and aBA2-CAR specifically targeting the second booster antigen, ... the n^{th} booster cells express the n^{th} booster antigen (BAn) and aBAn-i-CAR targeting the n-1 booster antigen, said n^{th} booster antigen BAn can be recognized by the therapeutic immune cells, and booster antigens of different grades are different from each other.

29. The adjuvant according to claim 28, **characterized in that** the n equals to any integer ranging from 1 to 10000000.

30. The adjuvant according to claim 28 or claim 29, **characterized in that** the booster cells of different stages can be the cells of the same type, or cells of different types, or a mixture of multiple types of cells.

31. A pharmaceutical composition, **characterized in that** the pharmaceutical composition comprises the adjuvant of any one of claims 1 to 9 or 28 to 30.

32. A pharmaceutical combination, **characterized in that** the pharmaceutical composition comprises the pharmaceutical composition according to claim 31, and the therapeutic immune cell according to any one of claims 14 to 27, wherein the therapeutic immune cells and the pharmaceutical composition are infused back in a single or consecutive multiple times at appropriate time points, so that the therapeutic immune cells can be activated by the adjuvant in the pharmaceutical composition and are quantitatively expanded, thus identifying and killing the target cells.

33. A method of treating cancer, **characterized in that** it comprises using the pharmaceutical composition according to claim 31 or the pharmaceutical combination of claim 32, wherein the pharmaceutical composition and the therapeutic immune cell are infused back in a single or consecutive multiple times at appropriate time points.


SEQUENCE LISTING

<110>  CHINEO MEDICAL TECHNOLOGY CO., LTD.

<120>  ADJUVANT CAPABLE OF BOOSTING THE EXAPANSION OF IMMUNE CELLS IN VIVO


<130>  BET 21M2502

<140>  EP 20755525.1
<141>  2020-02-11

<150>  CN 201910106097.7
<151>  2019-02-11

<160>  1

<170>  PatentIn version 3.5

<210>  1
<211>  1473
<212>  DNA
<213>  Artificial Sequence

<220>
<223>  EGFRviii CAR

<400>  1
   atggccctcc ctgtcaccgc cctgctgctt ccgctggctc ttctgctcca cgccgctcgg      60
   cccgagattc agctcgtgca atcgggagcg gaagtcaaga agccaggaga gtccttgcgg     120
   atctcatgca agggtagcgg ctttaacatc gaggattact acatccactg ggtgaggcag     180
   atgccgggga agggactcga atggatggga cggatcgacc cagaaaacga cgaaactaag     240
   tacggtccga tcttccaagg ccatgtgact attagcgccg atacttcaat caataccgtg     300
   tatctgcaat ggtcctcatt gaaagcctca gataccgcga tgtactactg tgctttcaga     360
   ggaggggtct actggggaca gggaactacc gtgactgtct cgtccggcgg aggcgggtca     420
   ggaggtggcg gcagcggagg aggagggtcc ggcggaggtg ggtccgacgt cgtgatgacc     480
   cagagccctg acagcctggc atacagcctg ggcgaaagag ctaccattaa ctgcaaatcg     540
   tcgcagagcc tgctggactc ggacggaaaa acgtacctca attggctgca gcaaaagcct     600
   ggccagccac cgaagcgcct tatctcactg gtgtcgaagc tggattcggg agtgcccgat     660
   cgcttctccg gctcgggatc gggtactgac ttcaccctca ctatctcctc gcttcaagca     720
   gaggatgttg ccgtctacta ctgctggcag ggaacccact ttccgggaac cttcggcgga     780
   gggacgaaag tggagatcaa gaccacgacg ccagcgccgc gaccaccaac accggcgccc     840
   accatcgcgt cgcagcccct gtccctgcgc ccagaggcgt gccggccagc ggcggggggc     900
   gcagtgcaca cgagggggct ggacttcgcc tgtgatatct acatctgggc gcccttggcc     960
   gggacttgtg gggtccttct cctgtcactg gttatcaccc tttactgcaa acggggcaga    1020
   aagaaactcc tgtatatatt caaacaacca tttatgagac cagtacaaac tactcaagag    1080
   gaagatggct gtagctgccg atttccagaa gaagaagaag gaggatgtga actgagagtg 1140
   aagttcagca ggagcgcaga cgcccccgcg tacaagcagg gccagaacca gctctataac 1200
   gagctcaatc taggacgaag agaggagtac gacgttttgg acaagagacg tggccgggac 1260
   cctgagatgg ggggaaagcc gagaaggaag aaccctcagg aaggcctgta caatgaactg 1320
   cagaaagata agatggcgga ggcctacagt gagattggga tgaaaggcga gcgccggagg 1380
   ggcaaggggc acgatggcct ttaccagggt ctcagtacag ccaccaagga cacctacgac 1440
   gcccttcaca tgcaggccct gccccctcgc taa 1473